(19) Europäisches Patentamt
European Patent Office
Office européen des brevets

(11) Veröffentlichungsnummer : **0 489 690 A1**

# (12) EUROPÄISCHE PATENTANMELDUNG

(21) Anmeldenummer : **91810926.5**

(22) Anmeldetag : **27.11.91**

(51) Int. Cl.$^5$ : **C07D 295/18,** A61K 31/495

(30) Priorität : **05.12.90 CH 3838/90**

(43) Veröffentlichungstag der Anmeldung :
**10.06.92 Patentblatt 92/24**

(84) Benannte Vertragsstaaten :
**AT BE CH DE DK ES FR GB GR IT LI LU NL SE**

(71) Anmelder : **CIBA-GEIGY AG**
**Klybeckstrasse 141**
**CH-4002 Basel (CH)**

(72) Erfinder : **Ferrini, Pier Giorgio, Dr.**
**Im Rehwechsel 22**
**CH-4102 Binningen (CH)**
Erfinder : **Burckhardt, Peter, Dr.**
**Kluserstrasse 40**
**CH-4054 Basel (CH)**

## (54) Substituierte N-Benzoyl-N'-(2-phenylethyl)-piperazine.

(57) N-Benzoyl-N'-(2-phenylethyl)-piperazine der Formel I,

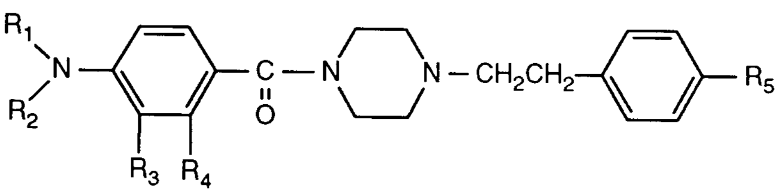

(I)

worin $R_1$, $R_2$, $R_3$, $R_4$ und $R_5$ wie in der Beschreibung definiert sind, und Salze davon, weisen analgetische Eigenschaften auf und können als analgetische Arzneimittelwirkstoffe verwendet werden. Sie werden beispielsweise hergestellt, indem man eine Verbindung der Formel II,

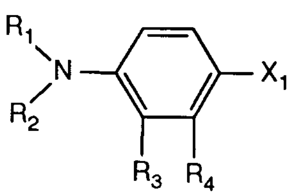

(II),

worin $X_1$ Carboxy oder reaktionsfähiges funktionell abgewandeltes Carboxy bedeutet, oder ein Salz davon, mit einer Verbindung der Formel III,

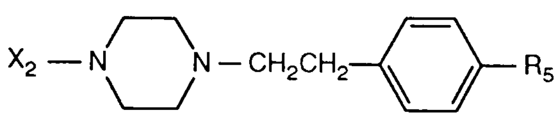

(III),

worin $X_2$ Wasserstoff bedeutet, umsetzt.

EP 0 489 690 A1

Die Erfindung betrifft neue N-Benzoyl-N′-(2-phenylethyl)-piperazine der Formel I

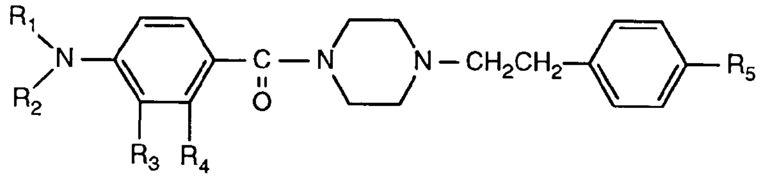 (I),

worin $R_1$ für Wasserstoff, Niederalkyl, Niederalkanoyl, Niederalkoxycarbonyl, Niederalkenyloxycarbonyl oder eine unsubstituierte oder durch Niederalkyl, Niederalkoxy, Halogen der Atomnummer bis und mit 35, Trifluormethyl, Nitro und/oder Amino substituierte Phenyloxycarbonyl- oder Phenylniederalkoxycarbonylgruppe steht, $R_2$ Wasserstoff oder gegebenenfalls durch ein Stickstoffatom unterbrochenes Niederalkyl bedeutet, $R_3$ und $R_4$ unabhängig voneinander Wasserstoff, Niederalkyl oder Halogen der Atomnummer bis und mit 35 bedeuten und $R_5$ Halogen der Atomnummer bis und mit 35 darstellt, mit der Massgabe, dass in Verbindungen der Formel I, in denen $R_5$ für Chlor steht und (a) $R_1$ Acetyl und $R_2$ Wasserstoff bedeuten, oder (b) $R_1$ und $R_2$ beide Methyl bedeuten, mindestens einer der Reste $R_3$ und $R_4$ von Wasserstoff verschieden ist, und Salze davon, Verfahren zur Herstellung dieser Verbindungen, diese enthaltende pharmazeutische Präparate und ihre Verwendung als Arzneimittelwirkstoffe.

Durch ein Stickstoffatom unterbrochenes Niederalkyl ist beispielsweise N-Mono- oder N,N-Diniederalkylaminoniederakyl.

Vor- und nachstehend sind unter niederen Resten und Verbindungen beispielsweise solche zu verstehen, die bis und mit 7, vorzugsweise bis und mit 4, Kohlenstoffatome (C-Atome) aufweisen.

Niederalkyl ist beispielsweise $C_1$-$C_7$-Alkyl, vorzugsweise $C_1$-$C_4$-Alkyl, wie insbesondere Methyl oder in zweiter Linie Ethyl, Propyl, Isopropyl oder Butyl, kann aber auch Isobutyl, Sekundärbutyl, Tertiärbutyl oder eine $C_5$-$C_7$-Alkyl-, wie Pentyl-, Hexyl- oder Heptylgruppe sein.

Niederalkanoyl ist beispielsweise $C_1$-$C_7$-Akanoyl, vorzugsweise $C_2$-$C_7$-Alkanoyl, wie Formyl, Acetyl, Propionyl, Butyryl, Isobutyryl oder Valeroyl.

Niederalkoxycarbonyl ist beispielsweise $C_1$-$C_7$-Alkoxycarbonyl, vorzugsweise $C_1$-$C_4$-Alkoxycarbonyl, wie Methoxy-, Ethoxy-, Propyloxy-, Isopropyloxy- oder Tertiärbutyloxycarbonyl, kann aber auch Isobutyloxy-, Sekundärbutyloxy- oder Butyloxycarbonyl oder eine $C_5$-$C_7$-Alkoxy-, wie Pentyloxy-, Hexyloxy- oder Heptyloxycarbonylgruppe sein.

Niederalkenyloxycarbonyl ist beispielsweise $C_2$-$C_7$-Alkenyloxycarbonyl, vorzugsweise $C_3$-$C_5$-Alkoxycarbonyl, wie Allyloxy- oder Methallyloxycarbonyl, kann aber auch eine Butenyloxy-, Pentenylox- oder Hexenyloxycarbonylgruppe sein.

Phenylniederalkoxycarbonyl ist beispielsweise Phenyl-$C_1$-$C_7$-alkoxycarbonyl, vorzugsweise Phenyl-$C_1$-$C_4$-alkoxycarbonyl, wie Benzyloxycarbonyl, 1- oder 2-Phenylethoxycarbonyl, 3-Phenylpropyloxycarbonyl oder 4-Phenylbutyloxycarbonyl, kann aber auch eine Phenyl-$C_5$-$C_7$-alkoxycarbonylgruppe sein.

N-Mono- oder N,N-Diniederalkylaminoniederalkyl trägt die N-Mono- oder N,N-Diniederalkylaminogruppe insbesondere in höherer als der $\alpha$-Stellung und ist beispeilsweise N-$C_1$-$C_4$-Alkylamino-$C_2$-$C_7$-alkyl, wie 2-Methylaminoethyl, 2-Ethylaminoethyl, 3-Methylaminopropyl, 3-Ethylaminopropyl oder 4-Methylaminobutyl, oder insbesondere N,N-Di-$C_1$-$C_4$-alkylamino-$C_1$-$C_7$-alkyl, wie 2-Dimethylaminoethyl, 2-Diethylaminoethyl, 3-Dimethylaminopropyl oder 4-Dimethylaminobutyl.

Halogen der Atomnummer bis und mit 35 ist beispielsweise Chlor oder Fluor, ferner Brom.

Salze von Verbindungen der Formel I sind insbesondere deren Säureadditionssalze, beispielsweise deren pharmazeutisch verwendbare Salze mit geeigneten Mineralsäuren, wie Halogenwasserstoffsäuren, Schwefelsäure oder Phosphorsäure, z.B. Hydrochloride, Hydrobromide, Sulfate, Hydrogensulfate oder Phosphate, Salze mit geeigneten aliphatischen oder aromatischen Sulfonsäuren oder N-substituierten Sulfaminsäuren, z.B Methansulfonate, Benzolsulfonate, p-Toluolsulfonate oder N-Cyclohexylsulfaminate (Cyclamate), oder Salze mit starken organischen Carbonsäuren, wie Niederalkancarbonsäuren oder gegebenenfalls ungesättigten oder hydroxylierten aliphatischen Dicarbonsäuren, z.B. Acetate, Oxalate, Malonate, Maleinate, Fumarate, Maleate, Tartrate oder Citronate.

Zur Isolierung oder Reinigung können auch pharmazeutisch ungeeignete Salze Verwendung finden. Zur therapeutischen Anwendung gelangen nur die pharmazeutisch verwendbaren, nicht-toxischen Salze, die deshalb bevorzugt sind.

Die Verbindungen der Formel I und ihre pharmazeutisch verwendbaren Salze weisen wertvolle pharmakologische Eigenschaften auf. Insbesondere zeigen sie ausgeprägte analgetische Wirkungen. Die analgetische Wirkung lässt sich beispielsweise anhand der Hemmung des durch Phenyl-p-benzochinon ausgelösten Writhingsyndroms der Maus beispielsweise nach Agent and Actions 23, 29-31 (1988) ab einer Dosis von etwa 0,1 mg/kg p.o. sowie an der Ratte anhand des experimentellen Essigsäure-Writhingsyndroms nach Pain Res. and Therap. 1, 517 (1976) ab ebenfalls etwa 0,1 mg/kg p.o. nachweisen.

Die Verbindungen der Formel I und ihre pharmazeutisch verwendbaren Salze können dementsprechend als analgetische Arzneimittelwirkstoffe zur Behandlung von Schmerzzuständen unterschiedlicher Genese, insbesondere als periphere Analgetika, verwendet werden.

Zusätzlich dazu weisen die Verbindungen der Formel I und ihre pharmazeutisch verwendbaren Salze eine ausgeprägte Hemmwirkung auf die Biosynthese von Interleukin-1 (IL-1). IL-1 gehört zur Klasse der proinflammatorischen Proteine und spielt beispielsweise bei der Prostaglandinsynthese, der Synthese neutraler Proteasen durch Fibroplasten, Synovialzellen und Chondrozyten, bei der Aktivierung von Endothelialzellen und bei der Induktion weiterer proinflammatorischer Zytokine, wie des α-Tumornekrosefaktors (TNF) und von Interleukin-6 (IL-6) eine wesentliche Rolle. Ferner regt es die Knochenresorption an, reguliert die Körpertemperatur von Warmblütern und reguliert u. a. die Entwicklung, Aktivierung, Differenzierung und Proliferation von Lymphozyten. Therapeutisch besonders wichtig ist die Hemmwirkung der Verbindungen der Formel I und ihrer pharmazeutisch verwendbaren Salze auf die Biosynthese von IL-1, TNF und IL-6. Diese kann *in vitro* beispielsweise an durch Lipopolysaccharide stimulierten (LPS-stimulierten) menschlichen Monozyten nach C. Rordorf-Adams et al., Drugs Exptl. Clin. Res. XV, 355-62 (1989) im Konzentrationsbereich ab etwa 1 μMol und *in vivo* in der Maus anhand der Hemmung der LPS-induzierten Bildung von Serumamyloid P (SAP) bei einer $ED_{50}$ von etwa 1 bis 15 mg/kg p.o. und an der Ratte anhand der Senkung des durch LPS erzeugten künstlichen Fiebers bei einer $ED_{50}$ von etwa 0,05 bis 3,5 mg/kg p.o. gezeigt werden.

Auf Grund dieser Eigenschaften sind die Verbindungen der Formel I und ihre pharmazeutisch verwendbaren Salze vorz vorzüglich geeignet zur therapeutischen Behandlung von Erkrankungen, bei denen eine übermässige Produktion von IL-1 durch monozytische bzw. makrophage Zellinien eine ursächliche oder verschlimmernde Rolle spielt, wie entzündliche und degenerative Gelenkerkrankungen, beispielsweise der rheumatoiden Arthritis, Osteoarthrosis, psoriatischen oder infektösen Arthritis, des Reitersyndroms, von Gicht und traumatischer Arthritis, und anderer akuter oder chronischer Entzündungen, beispielsweise von entzündlichen Darmerkrankungen, von Meningitis, Hauterkrankungen, wie Psoriasis, Pemphigus vulgaris und dergl., von allergischen Hautreaktionen und Autoimmunerkrankungen, wie Diabetes (Typ 1) und Thyroiditis.

Die Erfindung betrifft beispielsweise Verbindungen der Formel I, worin $R_1$ für Wasserstoff, Niederalkyl oder Niederalkanoyl steht, $R_2$ Wasserstoff oder Niederalkyl bedeutet, $R_3$ und $R_4$ unabhängig voneinander Wasserstoff, Niederalkyl oder Halogen der Atomnummer bis und mit 35 bedeuten und $R_5$ Halogen der Atomnummer bis und mit 35 darstellt, mit der Massgabe, dass in Verbindungen der Formel I, in denen $R_5$ für Chlor steht und (a) $R_1$ Acetyl und $R_2$ Wasserstoff bedeuten, oder (b) $R_1$ und $R_2$ beide Methyl bedeuten, mindestens einer der Reste $R_3$ und $R_4$ von Wasserstoff verschieden ist, und ihre Salze.

Die Erfindung betrifft in erster Linie Verbindungen der Formel I, worin $R_1$ Wasserstoff, $C_1$-$C_4$-Alkyl, wie Methyl oder Ethyl, $C_2$-$C_7$-Alkanoyl, wie Acetyl, Propionyl oder Valeroyl, $C_1$-$C_7$-Alkoxycarbonyl, vorzugsweise $C_1$-$C_4$-Alkoxycarbonyl, wie Methoxy-, Ethoxy-, Propyloxy-, Isopropyloxy- oder Tertiärbutyloxycarbonyl, $C_2$-$C_7$-Alkenyloxycarbonyl, vorzugsweise $C_3$-$C_5$-Alkoxycarbonyl, wie Allyloxy- oder Methallyloxycarbonyl, oder eine unsubstituierte oder durch Niederakyl, Niederalkoxy, Halogen der Atomnummer bis und mit 35, Trifluormethyl, Nitro und/oder Amino substituierte Phenyloxycarbonyl- oder Phenyl-$C_1$-$C_7$-alkoxycarbonyl-, vorzugsweise Phenyl-$C_1$-$C_4$-alkoxycarbonylgruppe, wie Benzyloxycarbonyl, 1- oder 2-Phenylethoxycarbonyl, 3-Phenylpropyloxycarbonyl oder 4-Phenylbutyloxycarbonyl, bedeutet, $R_2$ für Wasserstoff, $C_1$-$C_4$-Alkyl, wie Methyl oder Ethyl, N-$C_1$-$C_4$-Alkylamino-$C_2$-$C_7$-alkyl, wie 2-Methylaminoethyl, 2-Ethylaminoethyl, 3-Methylaminopropyl, 3-Ethylaminopropyl oder 4-Methylaminobutyl, oder insbesondere N,N-Di-$C_1$-$C_4$-alkylamino-$C_1$-$C_7$-alkyl, wie 2-Dimethylaminoethyl, 2-Diethylaminoethyl, 3-Dimethylaminopropyl oder 4-Dimethylaminobutyl, steht, einer der Reste $R_3$ und $R_4$ Wasserstoff, $C_1$-$C_4$-Alkyl, wie Methyl, oder Halogen der Atomnummer bis und mit 35, wie Fluor oder Chlor, und der andere Wasserstoff darstellt und $R_5$ Halogen der Atomnummer bis und mit 35, wie Fluor, Chlor oder Brom, bedeutet, mit der Massgabe, dass in Verbindungen der Formel I, in denen $R_5$ für Chlor steht und (a) $R_1$ Acetyl und $R_2$ Wasserstoff bedeuten, oder (b) $R_1$ und $R_2$ beide Methyl bedeuten, mindestens einer der Reste $R_3$ und $R_4$ von Wasserstoff verschieden ist, und ihre Salze, insbesondere ihre pharmazeutisch verwendbaren Salze.

Die Erfindung betrifft in erster Linie beispielsweise Verbindungen der Formel I, worin $R_1$ Wasserstoff, $C_1$-$C_4$-Alkyl, wie Methyl oder Ethyl, oder $C_2$-$C_7$-Alkanoyl, wie Acetyl, Propionyl oder Valeroyl, bedeutet, $R_2$ für Wasserstoff oder $C_1$-$C_4$-Alkyl, wie Methyl oder Ethyl, steht, einer der Reste $R_3$ und $R_4$ Wasserstoff, $C_1$-$C_4$-Alkyl, wie Methyl, oder Halogen der Atomnummer bis und mit 35, wie Fluor oder Chlor, und der andere Wasserstoff darstellt und $R_5$ Halogen der Atomnummer bis und mit 35, wie Fluor, Chlor oder Brom, bedeutet, mit der Massgabe,

dass in Verbindungen der Formel I, in denen $R_5$ für Chlor steht und (a) $R_1$ Acetyl und $R_2$ Wasserstoff bedeuten, oder (b) $R_1$ und $R_2$ beide Methyl bedeuten, mindestens einer der Reste $R_3$ und $R_4$ von Wasserstoff verschieden ist, und ihre Salze, insbesondere ihre pharmazeutisch verwendbaren Salze.

Die Erfindung betrifft insbesondere Verbindungen der Formel I, worin $R_1$ Wasserstoff, $C_2$-$C_7$-Alkanoyl, wie Acetyl, Propionyl oder Valeroyl, $C_1$ -$C_4$-Alkoxycarbonyl, wie Ethoxycarbonyl, $C_3$-$C_5$-Alkenyloxycarbonyl, wie Allyloxycarbonyl, Phenoxycarbonyl oder Phenyl-$C_1$-$C_4$-alkoxycarbonyl, wie Benzyloxycarbonyl, bedeutet, $R_2$ für Wasserstoff, $C_1$-$C_4$-Alkyl, wie Methyl oder Ethyl, oder N,N-Di-$C_1$-$C_4$-alkylamino-$C_1$-$C_7$-alkyl, wie 2-Dimethylaminoethyl, 2-Diethylaminoethyl, 3-Dimethylaminopropyl oder 4-Dimethylaninobutyl, steht, einer der Reste $R_3$ und $R_4$ Wasserstoff, $C_1$-$C_4$-Alkyl, wie Methyl, oder Halogen der Atomnummer bis und mit 35, wie Fluor oder Chlor, und der andere Wasserstoff darstellt und $R_5$ Halogen der Atomnummer bis und mit 35, wie Fluor, Chlor oder Brom, bedeutet, mit der Massgabe, dass in Verbindungen der Formel I, in denen $R_5$ für Chlor steht und $R_1$ Acetyl und $R_2$ Wasserstoff bedeuten, mindestens einer der Reste $R_3$ und $R_4$ von Wasserstoff verschieden ist, und ihre Salze, insbesondere ihre pharmazeutisch verwendbaren Salze.

Von besonderem Interesse sind Verbindungen der Formel I, worin $R_1$ $C_1$-$C_4$-Alkanoyl, wie Acetyl oder Propionyl, und $R_2$ Wasserstoff oder $C_1$-$C_4$-Alkyl, wie Methyl oder Ethyl, bedeutet oder $R_1$ und $R_2$ beide für Wasserstoff stehen, $R_3$ Wasserstoff oder $C_1$-$C_4$-Alkyl, wie Methyl, und $R_4$ Wasserstoff oder $R_3$ Wasserstoff und $R_4$ Halogen der Atomnummer bis und mit 35, wie Chlor oder Brom, darstellen und $R_5$ Halogen der Atomnummer bis und mit 35, wie Fluor, Chlor oder Brom, bedeutet, mit der Massgabe, dass in Verbindungen der Formel I, in denen $R_5$ für Chlor steht und $R_1$ Acetyl und $R_2$ Wasserstoff bedeuten, mindestens einer der Reste $R_3$ und $R_4$ von Wasserstoff verschieden ist, und ihre Salze, insbesondere ihre pharmazeutisch verwendbaren Salze.

Von ganz besonderem Interesse sind Verbindungen der Formel I, worin $R_1$ Wasserstoff oder $C_1$-$C_4$-Alkanoyl, wie Acetyl oder Propionyl, bedeutet, $R_2$ Wasserstoff darstellt, $R_3$ $C_1$-$C_4$-Alkyl, wie Methyl, und $R_4$ Wasserstoff oder $R_3$ Wasserstoff und $R_4$ Halogen der Atomnummer bis und mit 35, wie Chlor oder Brom, darstellen und $R_5$ Halogen der Atomnummer bis und mit 35, wie Fluor, Chlor oder Brom, bedeutet, mit der Massgabe, mit der Massgabe, dass in Verbindungen der Formel I, in denen $R_5$ für Chlor steht und $R_1$ Acetyl und $R_2$ Wasserstoff bedeuten, mindestens einer der Reste $R_3$ und $R_4$ von Wasserstoff verschieden ist, und ihre Salze, insbesondere ihre pharmazeutisch verwendbaren Salze.

Die Erfindung betrifft vorzugsweise einerseits Verbindungen der Formel I, worin $R_1$ Wasserstoff bedeutet, $R_2$ für Wasserstoff oder $C_1$-$C_4$-Alkyl, wie Methyl oder Ethyl, steht, einer der Reste $R_3$ und $R_4$ Wasserstoff, $C_1$-$C_4$-Alkyl, wie Methyl, oder Halogen der Atomnummer bis und mit 35, wie Fluor oder Chlor, und der andere Wasserstoff darstellt und $R_5$ Halogen der Atomnummer bis und mit 35, wie Fluor, Chlor oder Brom, bedeutet, und ihre Salze, insbesondere ihre pharmazeutisch verwendbaren Salze.

Die Erfindung betrifft vorzugsweise andererseits Verbindungen der Formel I, worin $R_1$ $C_2$-$C_7$-Alkanoyl, wie Acetyl, Propionyl oder Valeroyl, bedeutet, $R_2$ für Wasserstoff oder $C_1$-$C_4$-Alkyl, wie Methyl oder Ethyl, steht, $R_3$ Wasserstoff ist, $R_4$ für Wasserstoff oder Halogen der Atomnummer bis und mit 35, wie Chlor, steht und $R_5$ Halogen der Atomnummer bis und mit 35, wie Chlor oder Brom bedeutet, mit der Massgabe, dass in Verbindungen der Formel I, in denen $R_5$ für Chlor steht und $R_1$ Acetyl und $R_2$ Wasserstoff bedeuten, mindestens einer der Reste $R_3$ und $R_4$ von Wasserstoff verschieden ist, und ihre Salze, insbesondere ihre pharmazeutisch verwendbaren Salze.

Die Erfindung betrifft vor allem einerseits Verbindungen der Formel I, worin $R_1$ Wasserstoff bedeutet, $R_2$ für Wasserstoff oder $C_1$-$C_4$-Alkyl, wie Methyl oder Ethyl, steht, $R_3$ Wasserstoff oder $C_1$-$C_4$-Alkyl, wie Methyl, bedeutet, $R_4$ Wasserstoff, Fluor oder Chlor darstellt und $R_5$ Fluor, Chlor oder Brom bedeutet, und ihre Salze, insbesondere ihre pharmazeutisch verwendbaren Salze.

Die Erfindung betrifft vor allem andererseits Verbindungen der Formel I, worin $R_1$ $C_2$-$C_7$-Alkanoyl, wie Acetyl, Propionyl oder Valeroyl, oder Phenyl-$C_1$-$C_4$-alkoxycarbonyl, wie Benzyloxycarbonyl, bedeutet, $R_2$ für Wasserstoff, $C_1$-$C_4$-Alkyl, wie Methyl oder Ethyl, oder N,N-Di-$C_1$-$C_4$-alkylamino-$C_1$-$C_7$-alkyl, wie 2-Dimethylaminoethyl, steht, $R_3$ und $R_4$ für Wasserstoff stehen und $R_5$ Chlor bedeutet, mit der Massgabe, dass in Verbindungen der Formel I, in denen $R_5$ für Chlor steht und $R_1$ Acetyl und $R_2$ Wasserstoff bedeuten, mindestens einer der Reste $R_3$ und $R_4$ von Wasserstoff verschieden ist, und ihre Salze, insbesondere ihre pharmazeutisch verwendbaren Salze.

Die Erfindung betrifft vor allem andererseits beispielsweise Verbindungen der Formel I, worin $R_1$ $C_2$-$C_7$-Alkanoyl, wie Acetyl, Propionyl oder Valeroyl, bedeutet, $R_2$ für Wasserstoff oder $C_1$-$C_4$-Alkyl, wie Methyl oder Ethyl, steht, $R_3$ und $R_4$ für Wasserstoff stehen und $R_5$ Chlor bedeutet, mit der Massgabe, dass in Verbindungen der Formel I, in denen $R_5$ für Chlor steht und $R_1$ Acetyl und $R_2$ Wasserstoff bedeuten, mindestens einer der Reste $R_3$ und $R_4$ von Wasserstoff verschieden ist, und ihre Salze, insbesondere ihre pharmazeutisch verwendbaren Salze.

Die Erfindung betrifft in allererster Linie Verbindungen der Formel I, worin $R_1$ Wasserstoff bedeutet, $R_2$ für Wasserstoff oder in zweiter Linie für $C_1$-$C_4$-Alkyl, wie Methyl oder Ethyl, steht, $R_3$ Wasserstoff und $R_4$ Fluor oder

Chlor oder $R_3$ Methyl und $R_4$ Wasserstoff darstellt und $R_5$ Fluor, Chlor oder Brom bedeutet, und ihre Salze, insbesondere ihre pharmazeutisch verwendbaren Salze.

Die Erfindung betrifft namentlich die in den Beispielen genannten Verbindungen der Formel I und ihre Salze, insbesondere ihre pharmazeutisch verwendbaren Salze.

Das Verfahren zur Herstellung der Verbindungen der Formel I beruht auf an sich bekannten Methoden und ist dadurch gekennzeichnet, dass man

a) eine Verbindung der Formel II,

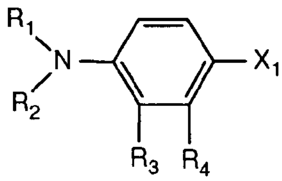

(II),

worin $X_1$ Carboxy oder reaktionsfähiges funktionell abgewandeltes Carboxy bedeutet, oder ein Salz davon, mit einer Verbindung der Formel III,

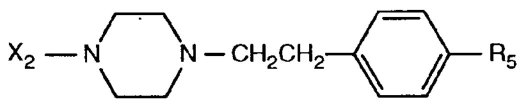

(III),

worin $X_2$ Wasserstoff oder eine Aminoschutzgruppe bedeutet, umsetzt, oder

b) eine Verbindung der Formel IV,

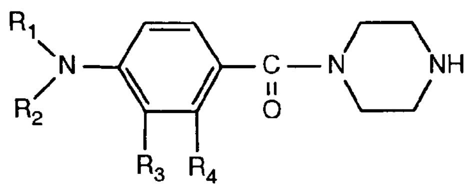

(IV)

oder ein Salz davon, mit einer Verbindung der Formel V,

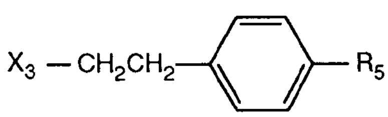

(V),

worin $X_3$ Hydroxy oder reaktionsfähiges verestertes Hydroxy bedeutet, umsetzt, oder

c) eine Verbindung der Formel VI,

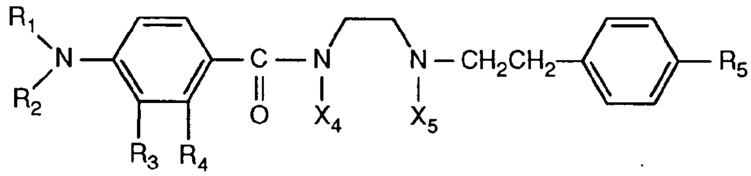

(VI)

worin einer der Reste $X_4$ und $X_5$ Wasserstoff und der andere eine Gruppe der Formel $-CH_2-CH_2-X_3$ (VIa) bedeutet, und $X_3$ für Hydroxy oder reaktionsfähiges verestertes Hydroxy steht, cyclisiert, oder

d) eine Verbindung der Formel VII,

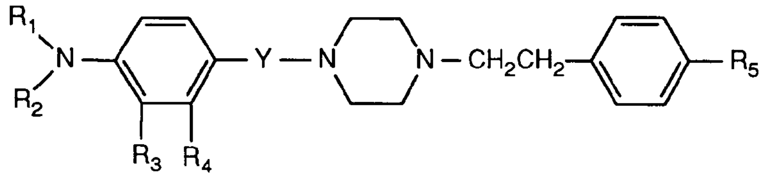

(VII),

worin Y eine zu Carbonyl oxidierbare Gruppe bedeutet, oxidiert und gewünschtenfalls eine verfahrensgemäss oder auf andere Weise erhältliche Verbindung in eine andere Verbindung der Formel I überführt, ein verfahrensgemäss erhältliches Isomerengemisch in die Komponenten auftrennt, eine verfahrensgemäss erhältliche freie Verbindung der Formel I in ein Salz überführt und/oder ein verfahrensmäss erhältliches Salz in die freie Verbindung der Formel I oder in ein anderes Salz überführt.

Die vor- und nachstehend beschriebenen Umsetzungen werden in an sich bekannter Weise durchgeführt, z.B. in Ab- oder üblicher Weise in Anwesenheit eines geeigneten Lösungs- oder Verdünnungsmittels oder eines Gemisches derselben, wobei man je nach Bedarf unter Kühlen, bei Raumtemperatur oder unter Erwärmen, z.B. in einem Temperaturbereich von etwa -78° bis zur Siedetemperatur des Reaktionsmediums, vorzugsweise von etwa -10° bis etwa 150°C, und, falls erforderlich, in einem geschlossenen Gefäss, unter Druck, in einer Inertgasatmosphäre und/oder unter wasserfreien Bedingungen arbeitet.

In den Ausgangsmaterialien kann das basische Zentrum z.B. in Form von Säureadditionssalzen, beispielsweise mit den vorstehend im Zusammenhang mit Salzen von Verbindungen der Formel I aufgeführten Säuren, vorliegen, während Ausgangsverbindungen der Formel II, worin $X_1$ Carboxy bedeutet, Salze mit Basen bilden können. Geeignete Salze mit Basen sind beispielsweise entsprechende Alkalimetall- oder Erdalkalimetallsalze, z.B. Natrium-, Kalium- oder Magnesiumsalze, pharmazeutisch verwendbare Übergangsmetallsalze, wie Zink- oder Kupfersalze, oder Salze mit Ammoniak oder organischen Aminen, wie cyclische Amine, wie Mono-, Di- bzw. Tri- hydroxy-$C_1$-$C_7$-alkylamine, Hydroxy-$C_1$-$C_7$-alkyl-$C_1$-$C_7$-alkyl-amine oder wie Polyhydroxy-$C_4$-$C_7$-alkylamine. Cyclische Amine sind z.B. Morpholin, Thiomorpholin, Piperidin oder Pyrrolidin. Als Mono-$C_1$-$C_7$-alkylamine kommen beispielsweise Ethyl- oder tert.- Butylamin, als Di-$C_1$-$C_7$-alkylamine beispielsweise Diethyl- oder Diisopropylamin, als Tri-$C_1$-$C_7$-alkylamine beispielsweise Trimethyl- oder Triethylamin in Betracht. Entsprechende Hydroxy-$C_1$-$C_7$-alkylamine sind z.B. Mono-, Di- bzw. Triethanolamine, und Hydroxy-$C_1$-$C_7$-alkyl-$C_1$-$C_7$-alkylamine sind z.B. N,N-Dimethylamino- oder N,N-Diethylaminoethanol, ferner Glucosamin als Polyhydroxy-$C_6$-alkylamin

Reaktionsfähiges funktionell abgewandeltes Carboxy $X_1$ bedeutet beispielsweise verestertes, in erster Linie reaktionsfähiges verestertes, Carboxy, anhydridisiertes Carboxy oder amidiertes Carboxy.

Verestertes Carboxy ist beispielsweise gegebenenfalls substituiertes $C_1$-$C_7$-Alkoxycarbonyl, wie Ethoxycarbonyl, vorzugsweise jedoch reaktionsfähiges verestertes Carboxy, beispielsweise gegebenenfalls, z.B. durch $C_1$-$C_7$-Alkoxy oder gegebenenfalls substituiertes Carbamoyl, zusätzlich aktiviertes Vinyloxycarbonyl, wie 1-$C_1$-$C_7$-Alkoxy-, z.B. 1-Ethoxyvinyloxycarbonyl, oder 2-(N-$C_1$-$C_7$-Alkyl-carbamoyl)-, z.B. 2-(N-Ethylcarbamoyl)-vinyloxycarbonyl, sowie gegebenenfalls, z.B. durch Nitro, Halogen, $C_1$-$C_7$-Alkansulfonyl oder Phenylazo, substituiertes Phenoxy- bzw. Thiophenoxycarbonyl, wie 4-Nitro-, 2,4,5-Trichlor-, Pentachlor-, 4-Methansulfonyl-, 4-Phenylazo-phenoxycarbonyl, Thiophenoxy- oder 4-Nitrothiophenoxycarbonyl, und ebenso aktiviertes, z.B. durch Cyano oder ferner gegebenenfalls verestertes Carboxy substituiertes, Methoxycarbonyl, insbesondere Cyanomethoxycarbonyl. Reaktionsfähiges verestertes Carboxy kann ebenso 1,1- oder 1,3-disubstituiertes 2-Isoureidocarbonyl, wie 1,1-Diniederalkyl-, 1,1-Diaryl- oder 1,1-Diaryl-$C_1$-$C_7$-alkyl-2-isoureidocarbonyl, z.B. 1,1-Diethyl-, 1,1-Diphenyl- oder 1,1-Dibenzyl-2-isoureidocarbonyl, oder 1,3-Dicycloalkyl-, z.B. 1,3-Dicyclohexyl-2-isoureido-carbonyl, oder N-$C_2$-$C_7$-Alkylenaminooxycarbonyl, wie N-Piperidinyl-oxycarbonyl, sowie N-Imido-oxycarbonyl, z.B. N-Succinimido-oxy- oder N-Phthalimido-oxycarbonyl, sein.

Unter anhydridisiertem Carboxy ist beispielsweise gegebenenfalls verzweigtes $C_1$-$C_7$-Alkoxycarbonyloxycarbonyl, wie Ethoxy- oder Isobutyloxycarbonyloxycarbonyl, Halogencarbonyl, wie Chlorcarbonyl, Azidocarbonyl, Halogenphosphoryloxycarbonyl, wie Dichlorphosphoryloxycarbonyl, oder gegebenenfalls, z.B. durch Halogen oder Aryl, substituiertes $C_1$-$C_7$-Alkanoyloxycarbonyl, wie Pivaloyloxy-, Trifluoracetyloxy- oder Phenylacetoxycarbonyl, zu verstehen.

Reaktionsfähiges amidiertes Carboxy ist beispielsweise gegebenenfalls, z.B. durch $C_1$-$C_7$-Alkyl, substituiertes 1-Imidazolyl- oder 1-Pyrazolylcarbonyl, wie 3,5-Dimethyl-pyrazolylcarbonyl.

Eine Aminoschutzgruppe $X_2$ ist beispielsweise Acyl, wie $C_1$-$C_7$-Alkanoyl, z.B. Formyl oder Acetyl, Halogencarbonyl, wie Chlorcarbonyl, ferner gegebenenfalls substituiertes Aryl- oder Heteroarylsulfonyl, wie 2-Pyridyl- oder 2-Nitrophenylsulfonyl.

Im Rahmen der vor- und nachstehenden Verfahrensbeschreibung bedeutet reaktionsfähiges verestertes

Hydroxy, z.B. $X_3$, sofern nicht abweichend definiert, insbesondere mit einer starken anorganischen Säure oder organischen Sulfonsäure verestertes Hydroxy, beispielsweise Halogen, wie Chlor, Brom oder Iod, Sulfonyloxy, wie Hydroxysulfonyloxy, Halogensulfonyloxy, z.B. Fluorsulfonyloxy, gegebenenfalls, z.B. durch Halogen, substituiertes $C_1$-$C_7$-Alkansulfonyloxy, z.B. Methan- oder Trifluormethansulfonyloxy, $C_3$-$C_7$-Cycloalkansulfonyloxy, z.B. Cyclohexansulfonyloxy, oder gegebenenfalls, z.B. durch $C_1$-$C_7$-Alkyl oder Halogen, substituiertes Benzolsulfonyloxy, z.B. p-Bromphenyl- oder p-Toluolsulfonyloxy.

Werden bei den vor- und nachstehend beschriebenen Umsetzungen beispielsweise Basen verwendet, so kommen, sofern nicht abweichend aufgeführt, beispielsweise Alkalimetallhydroxide, -hydride, -amide, -alkanolate, -carbonate, -triphenylmethylide, -di-$C_1$-$C_7$-alkylamide, -amino-$C_1$-$C_7$-alkylamide oder -$C_1$-$C_7$-alkylsilylamide, Naphthylamine, $C_1$-$C_7$-Alkylamine, basische Heterocyclen, Ammoniumhydroxide sowie carbocyclische Amine in Frage. Beispielhaft seien Lithiumhydroxid, Natriumhydroxid, -hydrid, -amid, -ethylat, Kalium-tert-butylat, -carbonat, Lithiumtriphenylmethylid, -diisopropylamid, Kalium-3-(aminopropyl)-amid, -bis-(trimethylsilyl)-amid, Dimethylaminonaphthalin, Di- oder Triethylamin, Pyridin, Benzyl-trimethylammoniumhydroxid, 1,5-Diaza-bicyclo[4.3.0]non-5-en (DBN) sowie 1,8-Diaza-bicyclo[5.4.0]undec-7-en (DBU) genannt.

Variante a):

Die verfahrensgemässe N-Acylierung wird in an sich bekannter Weise durchgeführt, erforderlichenfalls in Gegenwart eines, insbesondere basischen, Kondensationsmittels. Als Basen kommen beispielsweise Vertreter der vorstehend aufgeführten Basen in Frage. Häufig ist auch die Basizität der Verbindung der Formel III ausreichend.

Falls $X_1$ Carboxy bedeutet, werden z.B. primär die entsprechenden Ammoniumsalze gebildet, die durch Erwärmen oder Behandeln mit geeigneten Dehydratisierungsmitteln (als Kondensationsmittel), wie Carbodiimiden, z.B. N,N'-Diniederalkyl- oder N,N'-Dicycloakylcarbodiimid, wie N,N'-Diethyl-, N,N'-Diisopropyl- oder N,N'-Dicyclohexyl-carbodiimid, vorteilhaft unter Zusatz von N-Hydroxysuccinimid oder gegebenenfalls, z.B. durch Halogen, Niederalkoxy oder Niederalkyl, substituiertem 1-Hydroxy-benzotriazol oder N-Hydroxy-5-norbornen-2,3-dicarboxamid, sowie N,N-Carbonyldiimidazol, dehydratisiert werden können. Mit Carbodiimiden können intermediär z.B. auch die entsprechenden 1-Isoureidocarbonyl-Verbindungen gebildet werden. Als wasserbindende Kondensationsnittel können weiterhin N-Ethoxycarbonyl-2-ethoxy-1,2-dihydrochinolin, Phosphorylcyanamide bzw. -azide, wie Diethylphosphorylcyanamid oder Diphenylphosphorylazid, Triphenylphosphin-disulfid oder 1-Niederakyl-2-halogeno-piperidinium-halogenide, wie 1-Methyl-2-chlor- pyridinium-iodid, eingesetzt werden.

Die in dieser Verfahrensvariante verwendeten Ausgangsstoffe sind z.T. bekannt bzw. können nach an sich bekannten Verfahren hergestellt werden.

Zur Herstellung von Verbindungen der Formel II, worin $X_1$ gegebenenfalls substituiertes $C_1$-$C_7$-Alkoxycarbonyl bedeutet, kann man üblicherweise von der freien Säure ($X_1$ = Carboxy) oder einem Säureanhydrid ($X_1$ bedeutet z.B. Halogencarbonyl) ausgehen und diese beispielsweise mit dem entsprechenden, erforderlichenfalls in reaktionsfähiger Form vorliegenden, Alkohol, z.B. einem $C_1$-$C_7$-Alkylhalogenid, umsetzen. Die Herstellung von Verbindungen der Formel II, worin $X_1$ gegebenenfalls zusätzlich aktiviertes Vinyloxycarbonyl bedeutet, kann z.B. durch Umesterung eines $C_1$-$C_7$-Alkylesters mit Vinylacetat (Methode des aktivierten Vinylesters), durch Umsetzung der freien Säure von Verbindungen der Formel II mit Niederalkoxyacetylen (z. B. Ethoxyacetylen-Methode) oder, analog der Woodward-Methode, mit einem 1,2-Oxazoliumsalz erfolgen. Gegebenenfalls substituiertes Phenoxy- bzw. Thiophenoxycarbonyl aufweisende Verbindungen der Formel II können beispielsweise ausgehend von der freien Säure nach der Carbodiimid-Methode durch Umsetzen mit dem entsprechenden (Thio-)Phenol hergestellt werden.

Ebenfalls ausgehend von der freien Säure der Formel II können Verbindungen der Formel II, worin $X_1$ aktiviertes Methoxycarbonyl bzw. 1,1- oder 1,3-disubstituiertes 2-Isoureidocarbonyl darstellt, z.B. durch Umsetzung mit einem Halogen-, wie Chloracetonitril (Cyanmethylester-Methode) bzw. mit einem Carbodiimid oder Cyanamid (Carbodiimid- oder Cyanamid-Methode) erhalten werden. Die Herstellung von N-$C_2$-$C_7$-Alkylenamino-oxycarbonyl- bzw. N-Imido-oxycarbonyl-Verbindungen der Formel II kann beispielsweise bei Verwendung der freien Säure der Formel II aus entsprechenden N-Hydroxyverbindungen mit Hilfe von Carbodiimiden nach der Methode der aktivierten N-Hydroxyester erfolgen. Zur Herstellung von Verbindungen der Formel II, worin $X_1$ gegebenenfalls verzweigtes $C_1$-$C_7$-Alkoxycarbonyloxycarbonyl, Halogenphosphoryloxycarbonyl bzw. gegebenenfalls substituiertes $C_1$-$C_7$-Alkanoyloxycarbonyl bedeutet, kann beispielsweise von freier Säure der Formel II ausgegangen werden, die z.B. mit einem entsprechenden Halogenid, wie gegebenenfalls substituiertes $C_1$-$C_7$-Alkylkohlensäurehalogenid (Methode der gemischten O-Kohlensäureanhydride), Phosphoroxyhalogenid (z.B. Phosphoroxychlorid-Methode) oder gegebenenfalls substituiertes $C_1$-$C_7$-Alkanoylhalogenid (Methode der gemischten Carbonsäurehalogenide) behandelt werden kann. Azidocarbonylverbindungen der

Formel II sind beispielsweise durch Behandeln entsprechender Hydrazide mit salpetriger Säure zugänglich (Azid-Methode). Zur Herstellung von Verbindungen der Formel II, worin $X_1$ gegebenenfalls substituiertes 1-Imidazolyl-carbonyl bzw. 1-Pyrazolyl-carbonyl bedeutet, setzt man die freie Säure der Formel II z. B. mit Di-(1-imidazolyl)-carbonyl (Imidazolid-Methode) bzw. das betreffende Hydrazid z.B. mit einem entsprechenden 1,3-Diketon (Pyrazolid-Methode) um.

Variante b):

Der Rest $X_3$ bedeutet insbesondere reaktionsfähiges verestertes Hydroxy, bevorzugt Halogen, wie Chlor.

Die verfahrensgemässe N-Alkylierung wird in an sich bekannter Weise durchgeführt, erforderlichenfalls in Gegenwart einer, z.B, der vorstehend aufgeführten, Basen.

Die in dieser Verfahrensvariante verwendeten Ausgangsstoffe sind z.T. bekannt oder können in an sich bekannter Weise hergestellt werden.

So kann beispielsweise das Ausgangsmaterial der Formel IV hergestellt werden, indem man eine Verbindung der Formel

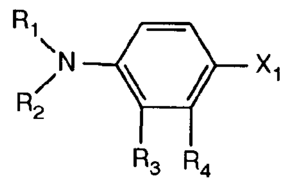

(II)

oder ein Salz davon, worin $X_1$ Carboxy oder reaktionsfähiges funktionell abgewandeltes Carboxy bedeutet, mit einer Verbindung der Formel

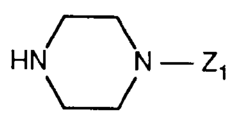

(IVa)

oder einem Salz davon, worin $Z_1$ Wasserstoff oder eine Aminoschutzgruppe, wie Benzyl, bedeutet, in der in Variante a) beschriebenen Weise umsetzt und gegebenenfalls die Aminoschutzgruppe, z.B. Benzyl durch übliche Hydrogenolyse, abspaltet.

Variante c):

Die verfahrensgemässe Cyclisierung (intramolekulare N-Alkylierung) wird in an sich bekannter Weise, erforderlichenfalls in Gegenwart eines, insbesondere basischen, Kondensationsmittels durchgeführt. Als Basen werden z.B. die vorstehend aufgeführten verwendet.

$X_3$ bedeutet dabei insbesondere reaktionsfähiges verestertes Hydroxy, bevorzugt Halogen, wie Chlor.

Das Ausgangsnaterial kann in an sich bekannter Weise hergestellt werden. Beispielsweise geht man von einer Verbindung der Formel

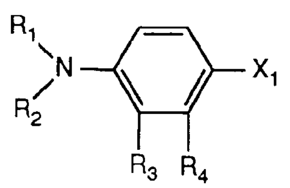

(II)

oder einem Salz davon aus, worin $X_1$ Carboxy oder reaktionsfähiges funktionell abgewandeltes Carboxy bedeutet, und setzt diese zunächst mit einer Verbindung der Formel

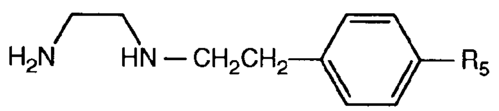

(VIb)

in Analogie zu Variante a) um. Im nächsten Reaktionsschritt wird die erhaltene Verbindung mit einer Verbindung der Formel $X_3$-$CH_2$-$CH_2$-$X_3$ (VIc) unter N-alkylierenden Bedingungen gemäss Variante b) umgesetzt.

Variante d):

Eine zu -CO- oxidierbare Gruppe Y steht in erster Linie für -$CH_2$-. Die Oxidation entsprechender Verbindungen der Formel VII erfolgt mit Hilfe eines geeigneten Oxidationsmittels, wobei bevorzugt gegebenenfalls, z.B. durch einen Phenylrest, substituierte Tetra-$C_1$-$C_4$-alkylammoniumpermanganate, in erster Linie Benzyltriethylammoniumpermanganat, verwendet wird.

Das Ausgangsmaterial der Formel VII wird in an sich bekannter Weise hergestellt. Beispielsweise geht man von einer Verbindung der Formel III aus, worin $X_2$ Wasserstoff bedeutet, und setzt diese unter den in Variante b) beschriebenen N-alkylierenden Bedingungen mit einer Verbindung der Formel

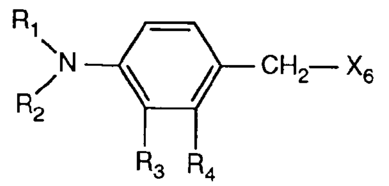

(VIIa)

um, wobei $X_6$ Hydroxy oder in erster Linie reaktionsfähiges verestertes Hydroxy, insbesondere Halogen, wie Chlor oder Brom, bedeutet.

Eine verfahrensgemäss oder anderweitig erhältliche erfindungsgemässe Verbindung kann in an sich bekannter Weise in eine andere erfindungsgemässe Verbindung übergeführt werden.

Erfindungsgemässe Verbindungen, in welchen einer der Reste $R_1$ und $R_2$ Wasserstoff und der andere Wasserstoff oder Niederalkyl ist, können in üblicher, beispielsweise in in der vorstehend unter Variante a) oder b) angegebenen Weise, N-mono- oder N,N-disubstituiert, d.h. durch Einführung einer Niederalkanoyl-, Niederalkoxycarbonyl-, Niederalkenyloxycarbonyl- oder unsubstituierten oder wie angegeben substituierten Phenyloxycarbonyl- oder Phenylniederalkoxycarbonylgruppe N-acyliert bzw. durch Einführung von Niederalkyl N-alkyliert, werden. So kann eine Verbindung der Formel I, worin $R_1$ Wasserstoff und $R_2$ Niederalkyl oder $R_1$ Niederalkanoyl, Niederalkoxycarbonyl, Niederalkenyloxycarbonyl oder unsubstituiertes oder wie angegeben substituiertes Phenyloxycarbonyl- oder Phenylniederalkoxycarbonyl und $R_2$ Wasserstoff bedeutet, zur entsprechenden N,N-Diniederalkylverbindung N-niederalkyliert oder zur entsprechenden N-Niederalkyl-N-niederalkanoyl-, N-Niederalkyl-N-niederalkoxycarbonyl-, N-Niederalkyl-N-niederalkenyloxycarbonyl, N-Niederalkyl-N-phenyloxycarbonyl- oder N-Niederalkyl-N-phenylniederakoxycarbonylverbindung N-niederakanoyliert bzw. N-niederalkyliert werden. Die Einführung einer Niederakanoyl-, Niederalkoxycarbonyl-, Niederalkenyloxycarbonyl- oder unsubstituierten oder wie angegeben substituierten Phenyloxycarbonyl- oder Phenylniederalkoxycarbonylgruppe erfolgt insbesondere durch Umsetzung mit einem entsprechenden Säureanhydrid, wie Säurechlorid, in Gegenwart eines basichen Kondensationsmittels, vorzugsweise Hünig'scher Base, in einem etherischen Lösungsmittel, wie einem cycloaliphatischen Ether, beispielsweise Tetrahydrofuran. Die N-Niederalkylierung erfolgt beispielsweise durch Umsetzung mit einem Niederalkylhalogenid oder Diniederalkylsulfat, kann aber auch analog der Leuckart-Wallach- (bzw. Eschweiler-Clarke)-Reaktion aus Carbonylverbindungen, z.B. unter Verwendung von Ameisensäure als Reduktionsmittel, reduktiv durchgeführt werden.

Erhaltene Salze können in an sich bekannter Weise in die freien Verbindungen umgewandelt werden, z.B. durch Behandeln mit einer Base, wie einem Alkalimetallhydroxid, einem Alkalimetallcarbonat oder -hydrogencarbonat, oder Ammoniak, oder einer anderen eingangs genannten salzbildenden Base bzw. mit einer Säure, wie einer Mineralsäure, z.B. mit Chlorwasserstoff, oder einer anderen eingangs genannten salzbildenden Säure.

Erhaltene Salze können in an sich bekannter Weise in andere Salze überführt werden, Säureadditionssalze z.B. durch Behandeln mit einem geeigneten Metallsalz, wie einem Natrium-, Barium- oder Silbersalz, einer anderen Säure in einem geeigneten Lösungsmittel, in welchem ein sich bildendes anorganisches Salz unlöslich

ist und damit aus dem Reaktionsgleichgewicht ausscheidet, und Basesalze durch Freisetzung der freien Säure und erneute Versalzung.

Die Verbindungen der Formel I, einschliesslich ihrer Salze, können auch in Form von Hydraten erhalten werden oder das zur Kristallisation verwendete Lösungsmittel einschliessen.

Infolge der engen Beziehung zwischen den neuen Verbindungen in freier Form und in Form ihrer Salze sind vorstehend und nachfolgend unter den freien Verbindungen und ihren Salzen sinn- und zweckgemäss gegebenenfalls auch die entsprechenden Salze bzw. freien Verbindungen zu verstehen.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes verwendet oder insbesondere unter den Reaktionsbedingungen bildet.

Die neuen Ausgangsstoffe, die speziell für die Herstellung der erfindungsgemässen Verbindungen entwickelt wurden, insbesondere die zu den eingangs als bevorzugt gekennzeicheten Verbindungen der Formel I führende Ausgangsstoffauswahl, die Verfahren zu ihrer Herstellung und ihre Verwendung als Zwischenprodukte bilden ebenfalls einen Gegenstand der Erfindung.

Die neuen Verbindungen der Formel I können z.B. in Form pharmazeutischer Präparate Verwendung finden, welche eine therapeutisch wirksame Menge der Aktivsubstanz, gegebenenfalls zusammen mit anorganischen oder organischen, festen oder flüssigen, pharmazeutisch verwendbaren Trägerstoffen enthalten, die sich zur enteralen, z. B. oralen, oder parenteralen Verabreichung eignen. So verwendet man Tabletten oder Gelatinekapseln, welche den Wirkstoff zusammen mit Verdünnungsmitteln, z. B. Lactose, Dextrose, Saccharose, Mannit, Sorbit, Cellulose und/oder Schmiermitteln, z.B. Kieselerde, Talk, Stearinsäure oder Salzen davon, wie Magnesium- oder Calciumstearat, und/oder Polyethylenglykol, aufweisen. Tabletten können ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxynethylcellulose und/oder Polyvinylpyrrolidon, und, wenn erwünscht, Sprengmittel, z.B. Stärken, Agar, Alginsäure oder ein Salz davon, z.B. Natriumalginat, und/oder Brausemischungen, oder Absorptionsmittel, Farbstoffe, Geschmacksstoffe und Süssmittel aufweisen. Ferner kann man die neuen Verbindungen der Formel I in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservierungs-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wirksame Stoffe enthalten können, werden in an sich bekannter Weise, z.B. mittels konventioneller Misch-, Granulier-, Dragier-, Lösungs- oder Lyophilisierungsverfahren hergestellt und enthalten von etwa 0,1% bis 100%, insbesondere von etwa 1% bis etwa 50%, Lyophilisate bis etwa 100% des Aktivstoffes.

Die Erfindung betrifft ebenfalls die Verwendung der Verbindungen der Formel I, vorzugsweise in Form von pharmazeutischen Präparaten. Die Dosierung kann von verschiedenen Faktoren, wie Applikationsweise, Spezies, Alter und/oder individuellem Zustand abhängen. Die täglich zu verabreichenden Dosen liegen bei oraler Applikation zwischen etwa 0,25 und etwa 10 mg/kg und für Warmblüter mit einem Körpergewicht von etwa 70 kg vorzugsweise zwischen etwa 20 mg und etwa 500 mg.

Die nachfolgen Beispiele dienen zur Illustration der Erfindung; Temperaturen sind in Celsiusgraden, Drucke in mbar angegeben.

Beispiel 1:

Eine bei -5° bis 0° gerührte Losung von 3.8 g p-Propionamidobenzoesäure in 30 ml wasserfreiem Dimethylformamid wird tropfenweise mit einer Lösung von 2 g Triethylamin in 5 ml wasserfreiem Dimethylformanid und anschliessend mit einer Lösung von 2. 15 g Chlorameisensäureethylester in 10 ml Methylenchlorid versetzt. Das Reaktionsgemisch wird 30 Minuten bei 0° bis 5° gerührt und danach wird eine Lösung von 4.4 g 1-[2-(4-Chlorphenyl)ethyl]-piperazin in 25 ml wasserfreiem Dimethylformamid dazugetropft. Die Suspension wird bei etwa 5° über Nacht gerührt und eingedampft. Der Rückstand wird mit 50 ml Wasser versetzt, mit 2 N Natronlauge alkalisch gestellt und mit Methylenchlorid ausgeschüttelt. Die vereinigten organischen Phasen werden mit Wasser gewaschen, über Natriumsulfat getrocknet, filtriert und eingedampft. Der Rückstand wird über Kieselgel chromatographiert und das erhaltene Öl aus Aceton/Diethylether kristallisiert. Man erhält so das 1-[4-(N-Propionylamino)benzoyl]-4-(2-(4-chlorphenyl)ethyl]-piperazin vom Smp. 131-132°.

Beispiel 2:

Eine bei Raumtemperatur gerührte Lösung von 3.3 g p-Propionamidobenzoesäure in 15 ml wasserfreiem Dimethylformamid wird mit 3 g N,N-Carbonyldiimidazol versetzt. Nach etwa 15 Minuten setzt sich die heftige Gasentwicklung ab. Man heizt auf 60° und nach 5 Minuten werden 3.8 g 1-[2-(4-Chlorphenyl)ethyl]-piperazin zugegeben. Man rührt 45 Minuten bei 80-85° Ölbadtemperatur. Die abgekühlte Lösung wird mit 2 N Natronlauge und Wasser versetzt und gerührt. Es kristallisiert 1-[4-(N-Propionylamino)benzoyl]-4-[2-(4-chlorphenyl)ethyl]-piperazin ab. Es wird abgenutscht und mit Wasser gewaschen. Es schmilzt bei 131 - 132°.

Beispiel 3:

37.2 g 1-(4-Nitrobenzoyl)-4-1-[2-(4-chlorphenyl)ethyl]-piperazin werden in 370 ml Tetrahydrofuran in Anwesenheit von 20 g Raney-Nickel bei Raumtemperatur reduziert. Die filtrierte Reaktionslösung wird eingedampft und das erhaltene Öl aus Isopropanol/Petrolether kristallisiert. Man erhält so 30 g 1-(4-Aminobenzoyl)-4-[2-(4-chlorphenyl)ethyl]-piperazin vom Smp. 111-112°.

Das 1-(4-Nitrobenzoyl)-4-(2-(4-chlorphenyl)ethyl]-piperazin wird in analoger Weise wie in Beispiel 2 beschrieben erhalten. Es schmilzt bei 109-110°.

Beispiel 4:

Eine Lösung von 2 g 1-(4-Aminobenzoyl)-4-[2-(4-chlorphenyl)ethyl]-piperazin (siehe Beispiel 3) in 20 ml Pyridin und 20 ml Valeriansäureanhydrid wird bei Raumtemperatur über Nacht stehen gelassen. Die Reaktionslösung wird auf 400 ml Wasser gegossen und mit 25 ml konzentrierter Natronlauge versetzt und 1 Stunde bei Raumtemperatur gerührt, wobei das Edukt auskristallisiert. Nach dem Abdekantieren der wässrigen Phase wird Methylenchlorid zugegeben und die organische Phase mit Wasser gewaschen, getrocknet, filtriert und eingedampft. Man erhält 2,9 g Öl, welches aus Diethylether kristallisiert wird. Das so erhaltene 1-[4-(N-Valeroylamino)benzoyl]-4-[2-(4-chlorphenyl)ethyl]-piperazin schmilzt bei 129-131°.

Beispiel 5:

Zu einer Suspension von 0.2g 50%-iger Suspension von Natriumhydrid in Mineralöl in 20 ml absolutem Dimethylformamid wird bei Raumtemperatur und unter Rühren eine Lösung von 1.8 g 1-[4-(N-Acetylamino)benzoyl]-4-(4-chlorphenylethyl)-piperazin in 20 ml absolutem Dimethylformamid zugetropft. Nach 45 Minuten wird eine Lösung von 0.7 g Methyljodid in 5 ml absolutem Dimethylformamid dazugetropft. Man rührt weiter über Nacht. Nach dem Abdestillieren von Dimethylformamid im Vakuum wird Wasser zugegeben. Es kristallisiert 1-[4-(N-Acetyl-N-methyl-amino)benzoyl]-4-[2-(4-chlorphenyl)ethyl]-piperazin vom Smp. 148-149° aus.

Das 1-[4-(N-Acetylamino)benzoyl]-4-(4-chlorphenylethyl)-piperazin wird in analoger Weise wie in Beispiel 2 beschrieben aus p-Acetamidobenzoesäure hergestellt. Das 1-[4-(N-Acetylamino)benzoyl]-4-(4-chlorphenylethyl)-piperazin kann auch aus 1-(4-Aminobenzoyl)-4-[2-(4-chlorphenyl)ethyl]-piperazin hergestellt werden, indem man 6.3 g 1-(4-Aminobenzoyl)-4-[2-(4-chlorphenyl)ethyl]-piperazin und 1.85 g Triethylamin in 70 ml Methylenchlorid löst und tropfenweise mit einer Lösung von 1.5 g Acetylchlorid in 10 ml Methylenchlorid versetzt. Die Reaktionslösung wird über Nacht stehen gelassen und danach eingedampft. Das erhaltene Öl wird in Ethanol aufgenommen, mit 20 ml 2 N Natronlauge versetzt und 4 Stunden bei Raumtemperatur gerührt. Ethanol wird dann im Vakuum entfernt, die Kristalle abgenutscht und aus Ethanol/Diethylether umkristallisiert Man erhält so das 1-[4-(N-Acetylamino)benzoyl]-4-(4-chlorphenylethyl)-piperazin vom Smp. 143-145°.

Beispiel 6:

1-[4-(N-Methyl-N-Propionyl-amino)benzoyl]-4-(2-(4-chlorphenyl)ethyl]-piperazin vom Smp. 138-139° kann in analoger Weise wie in Beispiel 5 beschrieben aus 2.85 g 1-[4-(N-Propionylamino)benzoyl]-4-[2-(4-chlorphenyl)ethyl]-piperazin (siehe Beispiel 2) hergestellt werden.

Beispiel 7:

In analoger Weise wie in Beispiel 3 beschrieben werden 35 g von 1-(2-Chlor-4-nitro-benzoyl)-4-[2-(4-chlorphenyl)ethyl]-piperazin zu 1-(4-Amino-2-chlorbenzoyl)-4-[2-(4-chlorphenyl)ethyl]-piperazin vom Smp. 175-176° reduziert.

Das Ausgangsmaterial wird aus 2-Chlor-4-nitro-benzoesäure über das Säurechlorid und nachfolgende

Umsetzung mit 1-[2-(4-Chlorphenyl)ethyl]-piperazin erhalten. Es schmilzt bei 115-117°

Beispiel 8:

3 g 1-(4-Amino-2-chlor-benzoyl)-4-[2-(4-chlorphenyl)ethyl]-piperazin werden in 30 ml Chloroform mit 10 ml Acetanhydrid 1 Stunde bei Raumtemperatur gerührt. Diese Lösung wird eingedampft und den Rückstand mit Wasser, 2 N Natronlauge und 20 ml Essigester versetzt und 15 Minuten gerührt. Nach dem Abnutschen und der Umkristallisation aus Ethanol/Petrolether man erhält 1-[4-(N-Acetylamino)-2-chlor-benzoyl]-4-[2-(4-chlorphenyl)ethyl]-piperazin vom Smp. 158-160°.

Beispiel 9:

1-[4-(N-Methylamino)benzoyl]-4-[2-(4-chlorphenyl)ethyl]-piperazin kann in analoger Weise wie in Beispiel 1 beschrieben aus 3 g p-(N-Methylamino)benzoesäure hergestellt werden. Aus diesem erhält man durch Behandeln mit einer Lösung von Chlorwasserstoff in Essigester das 1-[4-(N-Methylamino)benzoyl]-4-[2-(4-chlorphenyl)ethyl]-piperazin-hydrochlorid vom Smp. 156-158°.

1-[4-(N-Methylamino)benzoyl]-4-[2-(4-chlorphenyl)ethyl]-piperazin kann aber auch in analoger Weise wie in Beispiel 2 beschrieben aus 3.3 g p-(N-Methylamino)benzoesäure hergestellt werden. Das erhaltene viskose Öl wird über das Hydrochlorid gereinigt. Die zurückgewonnene Base wird an Kieselgel chromatographiert.

Beispiel 10:

In analoger Weise wie in Beispiel 3 beschrieben werden 20 g 1-(3-Methyl-4-nitro-benzoyl)-4-[2-(4-chlorphenyl)ethyl]-piperazin zu 1-(4-Amino-3-methyl-benzoyl)-4-[2-(4-chlorphenyl)ethyl]-piperazin vom Smp. 95-100° reduziert. Aus der Base erhält man das Hydrochlorid vom Smp. 250°.

Das Ausgangsmaterial wird aus m-Methyl-p-nitro-benzoesäure via Säurechlorid und Umsetzung mit 1-[2-(4-chlorphenyl)ethyl]-piperazin erhalten; es schmilzt bei 98°

Beispiel 11:

In analoger Weise wie in Beispiel 3 beschrieben werden 2.3 g von 1-(2-Fluor-4-nitro-benzoyl)-4-[2-(4-chlorphenyl)ethyl]-piperazin zu 1-(4-Amino-2-fluorbenzoyl)-4-[2-(4-chlorphenyl)ethyl]-piperazin vom Smp. 167-168° reduziert.

Das Ausgangsmaterial wird aus 2-Fluor-4-nitro-benzoesäure und Umsetzung mit 1-[2-(4-Chlorphenyl)ethyl]-piperazin in Anwesenheit von N,N-Carbonyldiimidazol erhalten. Es schmilzt bei 106-108°

Beispiel 12:

1-[4-(N-Ethyl-N-propionyl-amino)benzoyl]-4-[2-(4-chlorphenyl)ethyl]-piperazin vom Smp. 109- 110° kann in analoger Weise wie in Beispiel 5 beschrieben aus 1-[4-(N-Propionylamino)benzoyl]-4-[2-(4-chlorphenyl)ethyl]-piperazin (siehe Beispiel 2) hergestellt werden.

Beispiel 13:

0.8 g 1-[4-(N-Methylamino)-2-chlor-benzoyl]-4-[2-(4-chlorphenyl)ethyl]-piperazin (siehe Beispiel 8) wird in analoger Weise wie in Beispiel 5 beschrieben mit 0.3 g Methyljodid zu 1-[4-(N-Acetyl-N-methyl-amino)-2-chlorbenzoyl]-4-(2-(4-chlorphenyl)ethyl]-piperazin methyliert. Es schmilzt bei 162-163°.

Beispiel 14:

1.5 g 1-[4-Amino-2-chlor-benzoyl)-4-[2-(4-chlorphenyl)ethyl]-piperazin (siehe Beispiel 7) werden mit 0.26 g Acetaldehyd und 0.8 ml 5 N alkoholischer Salzsäure in 15 ml Methanol gelöst. Dazu werden 0.41 g $NaBH_4$ 85%-ig gegeben und 24 Stunden bei Raumtemperatur gerührt. Die Reaktionslösung wird mit Wasser versetzt, abdekantiert und in Methylenchlorid aufgenommen, getrocknet und eingedampft. Das ölige Material wird an Kieselgel chromatographiert und in Hydrochlorid übergeführt. Man erhält so 1-[4-(N-Ethylamino)-2-chlor-benzoyl]-4-[2-(4-chlorphenyl)ethyl]-piperazin-hydrochlorid vom Smp. 230°.

Beispiel 15:

0.8 g 1-[2-Chlor-4-N-propionyl-amino)benzoyl]-4-[2-(4-chlorphenyl)ethyl]-piperazin werden in analoger Weise wie in Beispiel 5 beschrieben mit 0.29 g Methyljodid zu 1-[4-(N-Methyl-N-propionyl-amino)-2-chlor-benzoyl]-4-[2-(4-chlorphenyl)ethyl]-piperazin methyliert und in das Hydrochlorid vom Smp. 135° übergeführt.

Das 1-[2-Chlor-4-N-propionyl-amino)benzoyl]-4-[2-(4-chlorphenyl)ethyl]-piperazin wird aus 1-[4-Amino-2-chlor-benzoyl)-4-[2-(4-chlorphenyl)ethyl]-pipera zin mit Propionsäureanhydrid in Chloroform hergestellt. Es schmilzt bei 128-130°.

Beispiel 16:

5 g 1-[3-Methyl-4-N-acetyl-amino)benzoyl]-4-[2-(4-chlorphenyl)ethyl]-piperazin werden in analoger Weise wie in Beispiel 5 beschrieben mit 0.8 ml Methyljodid zu 1-[4-(N-Acetyl-N-methyl-amino)-3-methyl-benzoyl]-4-[2-(4-chlorphenyl)ethyl]-piperazin methyliert und in das Hydrochlorid vom Smp. 195° übergeführt.

Das Ausgangsmaterial wird aus 1-(4-Amino-3-methyl-benzoyl)-4-[2-(4-chlorphenyl)ethyl]-piperazin (siehe Beispiel 10) mit Essigsäureanhydrid in Chloroform hergestellt. Es schmilzt bei 156-157°.

Beispiel 17:

4 g 1-(4-Amino-2-chlor-benzoyl)-4-[2-(4-chlorphenyl)ethyl]-piperazin (siehe Beispiel 7) werden in 500 ml wasserfreiem Dioxan gelöst und bei -15° tropfenweise mit 4 ml Chlorameisensäurebenzylester versetzt. Man rührt 1.5 Stunden bei Raumtemperatur. Das ausgefallene 1-[2-Chlor-4-(N-benzyloxycarbonylamino)benzoyl]-4-[2-(4-chlorphenyl)ethyl]-piperazin-hydrochlorid vom Smp. 195° wird abfiltriert und getrocknet.

Beispiel 18:

Das gemäss Beispiel 17 erhaltene 1-[2-Chlor-4-(N-benzyloxycarbonylamino)benzoyl]-4-[2-(4-chlorphenyl)ethyl]piperazin-hydrochlorid wird in analoger Weise wie in Beispiel 5 beschrieben mit 0.8 g Methyljodid methyliert. Man erhält 1-[2-Chlor-4-(N-benzyloxycarbonyl-N-methylamino)benzoyl]-4-[2(4-chlorphenyl)ethyl] -piperazin-hydrochlorid.

Beispiel 19:

Das gemäss Beispiel 18 erhaltene 1-[2-Chlor-4-(N-benzyloxycarbonyl-N-methyl-amino)benzoyl]-4-[2(4-chlorphenyl)ethyl]-piperazin-hydrochlorid wird in 15 ml Methanol gelöst und in Anwesenheit von 0.2 g Pd/C 10%-ig entbenzyliert. Man erhält so 1-[4-(N-Methylamino)-2-chlor-benzoyl]-4-[2-(4-chlorphenyl)ethyl]-piperazin vom Smp. 200°.

Beispiel 20:

1-[4-(N-Acetyl-N-ethyl-amino)benzoyl]-4-[2-(4-chlorphenyl)ethyl]-piperazin vom Smp. 133- 134° kann in analoger Weise wie in Beispiel 5 beschrieben aus 1-[4-(N-Acetylamino)benzoyl]-4-[2-(4-chlorphenyl)ethyl]-piper azin hergestellt werden.

Beispiel 21:

In analoger Weise wie in Beispiel 2 beschrieben kann aus 1.2 g 4-Methylamino-benzoesäure und 1.5 g 1-[2-(4-Fluorphenyl)ethyl]-piperazin 1-[4-(N-Methylamino)benzoyl]-4-[2-(4-fluorphenyl)ethyl]-piperazin hergestellt und als Hydrochlorid vom Smp. 207-208° kristallisiert werden.

Beispiel 22:

In analoger Weise wie in Beispiel 2 beschrieben wird aus 1.66 g 4-Methylamino-benzoesäure und 1.7 g 1-[2-(4-Bromphenyl)ethyl]-piperazin 1-[4-(N-Methylamino)benzoyl]-4-[2-(4-bromphenyl)ethyl]-piperazin vom Smp. 92-93.5° hergestellt.

Beispiel 23:

In analoger Weise wie in Beispiel 2 beschrieben wird aus 1.65 g 4-Ethylaminobenzoesäure und 2.2 g 1-[2-(4-chlorphenyl)ethyl]-piperazin 1-[4-(N-Ethylamino)benzoyl]-4-[2-(4-chlorphenyl)ethyl]-piperazin vom Smp. 99-101° hergestellt.

Beispiel 24:

In analoger Weise wie in Beispiel 2 beschrieben wird aus 4.7 g 4-Nitro-3-brom-benzoesäure und 1-[2-(4-chlorphenyl)ethyl]-piperazin 1(-4-Nitro-3-brom-benzoyl)-4-[2-(4-chlorphenyl)ethyl]-piperazin hergestellt und wie in Beispiel 3 beschrieben in Anwesenheit von Raney-Nickel zu 1-(4-Amino-3-brom-benzoyl)-4-[2-(4-chlorphenyl)ethyl]-piperazin vom Smp. 120-123° reduziert.

Beispiel 25:

In analoger Weise wie in Beispiel 5 beschrieben wird aus 1-(4-Acetylaminobenzoyl)-4-[2-(4-bromphenyl)ethyl]-piperazin und 1.6 g Ethyljodid 1-[4-(N-Acetyl-N-ethyl-amino)benzoyl]-4-[2-(4-bromphenyl)ethyl]-piperazin vom Smp. 131-131.5° hergestellt.

Das Ausgangsmaterial wird in analoger Weise wie in Beispiel 2 beschrieben aus 4-Acetamidobenzoesäure und 1-[2-(4-Bromphenyl)ethyl]-piperazin vom Smp. 193-194° erhalten.

Beispiel 26:

Zu einer Lösung von 1.03 g 1-[4-N-Methylaminobenzoyl]-4-[2-(4-chlorphenyl)ethyl]-piperazin in 15 ml wasserfreiem Tetrahydrofuran wird bei Raumtemperatur und unter Rühren 0.45 g N-Ethyl-diisopropylamin zugetropft. Anschliessend werden 1.69 ml 33%ige Chlorameisensäurebenzylester in Toluol zugetropft. Die Reaktionslösung wird 30 Minuten bei Raumtemperatur gerührt und danach am Rotationsverdampfer eingedampft. Der ölige Rückstand wird in Essigester aufgenommen und mit Wasser und 5%iger Sodalösung gewaschen, mit Magnesiumsulfat getrocknet, filtriert und eingedampft. Nach dem Digerieren mit Ether kristallisiert das 1-[4-(N-Benzyloxycarbonyl-N-methylamino)benzoyl]-4-[2-(4-chlorphenyl)ethyl]-piperazin vom Smp. 78-79°.

Beispiel 27:

In analoger Weise wie im Beispiel 26 beschrieben werden hergestellt:

aus 0.70 g 1-[4-(N-Butylamino)benzoyl]-4-[2-(4-chlorphenyl)ethyl]-piperazin erhält man 1-[4-(N-Benzyloxycarbonyl-N-butylamino)benzoyl]-4-[2-(4-chlorphenyl)ethyl]-piperazin vom Smp. 146-148°.

aus 1.06 g 1-[4-Amino-3-methyl-benzoyl]-4-[2-(4-chlorphenyl)ethyl]-piperazin erhält man 1-[4-(N-Benzyloxycarbonylamino)-3-methyl-benzoyl]-4-[2-(4-chlorphenyl)ethyl]-piperazin vom Smp. 113-115°.

aus 0.74 g 1-[4-(N-Ethylamino)benzoyl]-4-[2-(4-bromphenyl)ethyl]-piperazin erhält man 1-[4-(N-Benzyloxycarbonyl-N-ethylamino)benzoyl]-4-[2-(4-bromphenyl)ethyl]-piperazin vom Smp. 61-62°.

aus 0.74 g 1-[4-(N-Methylamino)benzoyl]-4-[2-(4-bromphenyl)ethyl]-piperazin erhält man 1-[4-(N-Benzyloxycarbonyl-N-methylamino)benzoyl]-4-[2-(4-bromphenyl)ethyl]-piperazin vom Smp. 81-82°.

aus 1.04 g 1-[4-Aminobenzoyl]-4-[2-(4-fluorphenyl)ethyl]-piperazin erhält man 1-[4-(N-Benzyloxycarbonylamino)benzoyl]-4-[2-(4-fluorphenyl)ethyl]-piperazin vom Smp. 158-159°.

aus 0.60 g 1-[4-Amino-2-fluor-benzoyl]-4-[2-(4-chlorphenyl)ethyl]-piperazin erhält man 1-[4-(N-Benzyloxycarbonylamino)-2-fluor-benzoyl]-4-[2-(4-chlorphenyl)ethyl]-piperazin vom Smp. 140-141°.

aus 0.45 g 1-[4-Amino-2-brom-benzoyl]-4-[2-(4-chlorphenyl)ethyl]-piperazin erhält man 1-[4-(N-Benzyloxycarbonylamino)-2-brom-benzoyl]-4-[2-(4-chlorphenyl)ethyl]-piperazin vom Smp. 128-130°.

aus 0.62 g 1-[4-(N-{2-Dimethylaminoethyl}amino)benzoyl]-4-[2-(4-chlorphenyl)ethyl]-piperazin erhält man 1-[4-(N-Benzyloxycarbonyl-N-{2-dimethylaminoethyl}amino)benzoyl]-4-[2-(4-chlorphenyl)ethyl]-piperazin-hydroch lorid vom Smp. 133-136°. Das Ausgangsmaterial kann in analoger Weise wie in Beipiel 14 beschrieben aus 1-[4-(Amino)benzoyl]-4-[2-(4-chlorphenyl)ethyl]-piperazin und 2-Dimethylaminoacetaldehyd hergestellt werden.

Beispiel 28:

8 g 1-[4-Aminobenzoyl]-4-[2-(4-chlorphenyl)ethyl]-piperazin werden in 200 ml Dioxan gelöst. Dazu werden

bei 15° 8.7 g Chlorameisensäurebenzylester als 50%ige Toluollösung zugetropft. Nach 2 Stunden bei Raumtemperatur wird das Reaktionsgemisch mit Wasser verdünnt, Dioxan wegdestilliert und die wässrige Phase mit 2 N Natronlauge alkalisch gestellt und mit Essigester ausgeschüttelt. Das aus dem Essigester gewonnene Öl wird aus Alkohol und Ether kristallisiert. Man erhält so 1-[4-(N-Benzyloxycarbonylamino)benzoyl]-4-[2-(4-chlorphenyl)ethyl]-piperazin vom Smp. 149-150°.

Beispiel 29:

90 g 1-[4-(N-Acetylamino)benzoyl]-4-[2-(4-chlorphenyl)ethyl]-piperazin und 2.8 g 4-Dimethylaminopyridin werden in 800 ml Acetonitril suspendiert. Unter Aussenkühlung wird dazu eine Lösung von 62 g BOC-anhydrid in 100 ml Acetonitril innerhalb 20 Minuten zugetropft. Die Suspension wird über Nacht bei Raumtemperatur gerührt. Danach werden 47.4 g 2-Diethylaminoethylamin in 50 ml Acetonitril zugetropft (das intermediär auftretende 1-[4-(N-Butyloxycarbonyl-N-acetylamino)benzoyl]-4-[2-(4-chlor phenyl)ethyl]-piperazin wird verseift nach L. Grehn et al., Acta Chem. Scand. B41, 18-23, 1987 verseift) Die entstandene klare Lösung wird über Nacht gerührt. Die Reaktionslösung wird eingedampft. Der ölige Rückstand wird mit Essigester ausgeschüttelt, eingedampft und aus Ether kristallisiert. Man erhält so 1-[4-(N-Tertiärbutyloxycarbonylamino)benzoyl]-4-[2-(4-chlorphenyl)ethyl]-piperazin vom Smp. 127-128°.

Beispiel 30:

1 g 1-[4-Aminobenzoyl]-4-[2-(4-chlorphenyl)ethyl]-piperazin und 0.4 g Hünigbase (= N-Ethyldiisopropylamin) werden in 15 ml Tetrahydrofuran gelöst. Dazu wird eine Lösung von 0.75 g Chlorameisensäure-4-nitrobenzylester in 5 ml Tetrahydrofuran getropft. Man rührt 3.5 Stunden bei Raumtemperatur und 2 Stunden bei 55° Badtemperatur. Die Lösung wird eingedampft, den Rückstand in Methylenchlorid aufgenommen und mit Wasser gewaschen. Man erhält aus Alkohol farblose Kristalle von 1-[4-(N-4-Nitrobenzyloxycarbonylamino)benzoyl]-4-[2-(4-chlorphenyl)ethyl]-piperazin vom Smp. 146-148°.

Beispiel 31:

In analoger Weise wie in Beispiel 30 beschrieben werden hergestellt:
aus 1 g 1-[4-Aminobenzoyl]-4-[2-(4-chlorphenyl)ethyl]-piperazin 1-[4-(N-Phenyloxycarbonylamino)benzoyl]-4-[2-(4-chlorphenyl)ethyl]-piperazin vom Smp. 174-175°;
aus 1 g 1-[4-Aminobenzoyl]-4-[2-(4-chlorphenyl)ethyl]-piperazin 1-[4-(N-Allyloxycarbonylamino)benzoyl]-4-[2-(4-chlorphenyl)ethyl]-piperazin vom Smp. 136-138°;
aus 1 g 1-[4-Aminobenzoyl]-4-[2-(4-chlorphenyl)ethyl]-piperazin 1-[4-(N-Ethoxycarbonylamino)benzoyl]-4-[2-(4-chlorphenyl)ethyl]-piperazin vom Smp. 161-162°.

Beispiel 32:

3.0 g 1-(4-Amino-2-chlor-benzoyl)-4-[2-(4-chlorphenyl)ethyl]-piperazin werden mit 10 ml Buttersäureanhydrid in 50 ml Chloroform 2h bei Raumtemperatur (RT) gerührt. Die Lösung wird eingedampft, der Rückstand mit NaOH alkalisch gestellt und mit Essigester extrahiert. Die organische Phase wird eingedampft und der Rückstand aus Toluol umkristallisiert; so erhält man das 1-(4-N-Butyrylamino-2-chlor-benzoyl)-4-[2-(4-chlorphenyl)ethyl]-piperazin, Smp. 135-137°.

Beispiel 33:

1.7 g 1-[4-(N-Acetylamino)benzoyl]-4-[2-(4-chlorphenyl)ethyl]-piperazin, gelöst in 20 ml DMF, werden zu einer Suspension von 0.23 g 50% NaH in 20 ml DMF gegeben. Nach 1.5h Rühren bei RT werden 0.97 g n-Propyliodid, gelöst in 5 ml DMF, hinzugetropft. Man lässt über Nacht bei RT rühren, destilliert das DMF im Vakuum ab, versetzt mit Wasser und extrahiert mit Essigester. Die organische Phase wird eingedampft und der Rückstand aus Ether/Petrolether umkristallisiert; man erhält so das 1-[4-(N-Acetyl-N-propylamino)benzoyl]-4-[2-(4-chlorphenyl)ethyl]-piperazin, Smp. 112-113°.

Beispiel 34:

Zu einer Lösung von 1.9 g 4-Butylaminobenzoesäure in 25 ml DMF werden 1.7 g 1,1-Carbonyldiimidazol gegeben. Zunächst wird 5 min bei RT gerührt, dann 15 min bei 100°; man lässt etwas abkühlen und gibt unter

Rühren 2.2 g N-[2-(4-Chlorphenethyl)]-piperazin hinzu. Man rührt 1h bei 100°, lässt abkühlen und giesst das Gemisch auf 150 ml Wasser. Das Produkt wird mit Dichlormethan extrahiert und die organische Phase getrocknet und eingedampft. Der ölige Rückstand wird über 60 g Kieselgel mit dem Eluiermittel Dichlormethan/Aceton 9:1 chromatographiert. Das erhaltene viskose Oel wird bei 70° im Hochvakuum getrocknet. Nach einer Woche kristallisiert das Produkt, 1-[4-(N-Butylamino)benzoyl]-4-[2-(4-chlorphenyl)ethyl]-piperazin, aus, Smp. 79-80°.

Beispiel 35:

1.6 g 1-(4-Acetylaminobenzoyl)-4-[2-(4-bromphenyl)ethyl]-piperazin, gelöst in 15 ml DMF, werden analog Beispiel 5 zunächst mit 0.2 g NaH in 10 ml DMF und dann mit 0.6 g Methyliodid in 5 ml DMF umgesetzt. Das Rohprodukt wird aus Ether umkristallisiert. Man erhält so 1-[4-(N-Acetyl-N-methylamino)benzoyl]-4-[2-(4-bromphenyl)ethyl]-piperazin vom Smp. 161-162°.

Beispiel 36:

Tabletten, enthaltend je 50 mg 1-(4-Amino-2-chlor-benzoyl)-4-[2-(4-chlorphenyl)ethyl]-piperazin oder ein Salz, z.B. das Hydrochlorid, davon können wie folgt hergestellt werden:

Zusammensetzung (10000 Tabletten)

| | |
|---|---|
| Wirkstoff | 500,0 g |
| Lactose | 500,0 g |
| Kartoffelstärke | 352,0 g |
| Gelatine | 8,0 g |
| Talk | 60,0 g |
| Magnesiumstearat | 10,0 g |
| Siliciumdioxid (hochdispers) | 20,0 g |
| Ethanol | q. s. |

Der Wirkstoff wird mit der Lactose und 292 g Kartoffelstärke vermischt, die Mischung mit einer ethanolischen Lösung der Gelatine befeuchtet und durch ein Sieb granuliert. Nach dem Trocknen mischt man den Rest der Kartoffelstärke, das Magnesiumstearat, das Talk und das Siliciumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

Beispiel 37:

Gelatinesteckkapseln, enthaltend 100 mg Wirkstoff, z. B. 1-(4-Amino-2-chlor-benzoyl)-4-[2-(4-chlorphenyl)ethyl]-piperazin oder ein Salz, z.B. das Hydrochlorid, davon, können z.B. folgendermassen hergestellt werden:

Zusammensetzung (für 1000 Kapseln)

| | |
|---|---|
| Wirkstoff | 100,0 g |
| Lactose | 250,0 g |
| mikrokristalline Zellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem lyophilisierten Wirkstoff hinzugesiebt. Beide Komponenten werden innig vermischt. Dann wird zunächst die Lactose durch ein Sieb mit einer Maschenweite von 0,6 mm und dann die mikrokristalline Zellulose durch ein Sieb mit einer Maschenweite von 0,9 mm hinzugesiebt. Daraufhin wird erneut 10 Minuten innig gemischt. Zuletzt wird das Magnesiumstearat durch ein Sieb mit einer Maschenweite von 0,8 mm hinzugesiebt. Nach 3- minütigem weiteren Mischen werden je 390 mg der erhaltenen Formulierung in Gelatinesteckkapseln der Grösse 0 abgefüllt.

Beispiel 38:

Lacktabletten, enthaltend je 100 mg 1-(4-Amino-2-chlor-benzoyl)-4-[2-(4-chlorphenyl)ethyl]-piperazin oder ein Salz, z.B. das Hydrochlorid, davon können wie folgt hergestellt werden:

Zusammensetzung (für 1000 Lacktabletten)

| | |
|---|---|
| Wirkstoff | 100,0 g |
| Lactose | 100,0 g |
| Maisstärke | 70,0 g |
| Talk | 8,5 g |
| Calciumstearat | 1,5 g |
| Hydroxypropylmethylcellulose | 2,36 g |
| Schellack | 0,64 g |
| Wasser | q. s. |
| Methylenchlorid | q.s. |

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat zugegeben und mit dem Granulat vermischt. Das Gemisch wird zu Tabletten (Gewicht: 280 mg) verpresst und diese mit einer Lösung der Hydroxypropyl-methylcellulose und des Schellacks in Methylenchlorid lackiert; Endgewicht der Lacktablette: 283 mg.

Beispiel 39:

Eine 0,2%-ige Injektions- oder Infusionslösung von 1-(4-Amino-2-chlor-benzoyl)-4-[2-(4-chlorphenyl)ethyl]-piperazin oder eines Salzes, z.B des Hydrochlorides, davon, kann beispielsweise folgendermassen hergestellt werden:

Zusammensetzung (für 1000 Ampullen)

| | |
|---|---|
| Wirkstoff | 5,0 g |
| Natriumchlorid | 22,5 g |
| Phosphatpuffer pH= 7.4 | 300,0 g |
| entmineralisiertes Wasser | ad 2500,0 ml |

Der Wirkstoff und das Natriumchlorid werden in 1000 ml Wasser gelöst und durch ein Mikrofilter filtriert. Man versetzt mit der Pufferlösung und füllt mit Wasser auf 2500 ml auf. Zur Herstellung von Dosiseinheitsformen werden je 1,0 oder 2,5 ml in Glasampullen abgefüllt, die dann je 2,0 bzw. 5,0 mg Wirkstoff enthalten.

Beispiel 40:

In analoger Weise wie in Beispiel 36 bis 39 beschrieben, können auch Tabletten, enthaltend jeweils 25 mg einer anderen der in den Beispielen 1 bis 35 genannten Verbindungen hergestellt werden.

## Patentansprüche

**1.** Ein neues N-Benzoyl-N'-(2-phenylethyl)-piperazin der Formel I

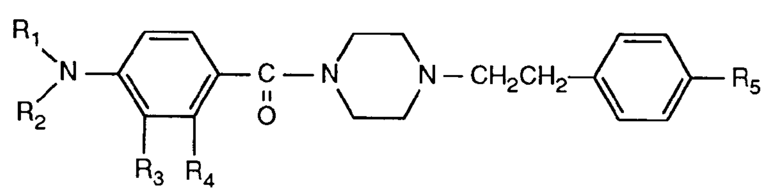 (I),

worin $R_1$ für Wasserstoff, Niederalkyl, Niederalkanoyl, Niederalkoxycarbonyl, Niederalkenyloxycarbonyl oder eine unsubstituierte oder durch Niederalkyl, Niederalkoxy, Halogen der Atomnummer bis und mit 35, Trifluormethyl, Nitro und/oder Amino substituierte Phenyloxycarbonyl- oder Phenylniederalkoxycarbonylgruppe steht, $R_2$ Wasserstoff oder gebebenenfalls durch ein Stickstoffatom unterbrochenes Niederalkyl bedeutet, $R_3$ und $R_4$ unabhängig voneinander Wasserstoff, Niederalkyl oder Halogen der Atomnummer bis und mit 35 bedeuten und $R_5$ Halogen der Atomnummer bis und mit 35 darstellt, mit der Massgabe, dass in Verbindungen der Formel I, in denen $R_5$ für Chlor steht und (a) $R_1$ Acetyl und $R_2$ Wasserstoff bedeu-

ten, oder (b) $R_1$ und $R_2$ beide Methyl bedeuten, mindestens einer der Reste $R_3$ und $R_4$ von Wasserstoff verschieden ist, oder ein Salz davon.

2. Eine Verbindung gemäss Anspruch 1 der Formel I, worin $R_1$ für Wasserstoff, Niederalkyl oder Niederalkanoyl steht, $R_2$ Wasserstoff oder Niederalkyl bedeutet, $R_3$ und $R_4$ unabhängig voneinander Wasserstoff, Niederalkyl oder Halogen der Atomnummer bis und mit 35 bedeuten und $R_5$ Halogen der Atomnummer bis und mit 35 darstellt, mit der Massgabe, dass in Verbindungen der Formel I, in denen $R_5$ für Chlor steht und (a) $R_1$ Acetyl und $R_2$ Wasserstoff bedeuten, oder (b) $R_1$ und $R_2$ beide Methyl bedeuten, mindestens einer der Reste $R_3$ und $R_4$ von Wasserstoff verschieden ist, oder ein Salz davon.

3. Eine Verbindung gemäss Anspruch 1 der Formel I, worin $R_1$ Wasserstoff, $C_1$-$C_4$-Alkyl, $C_2$-$C_7$-Alkanoyl, $C_1$-$C_7$-Alkoxycarbonyl, $C_2$-$C_7$-Alkenyloxycarbonyl oder eine unsubstituierte oder durch Niederalkyl, Niederalkoxy, Halogen der Atomnummer bis und mit 35, Trifluormethyl, Nitro und/oder Amino substituierte Phenyloxycarbonyl- oder Phenyl-$C_1$-$C_7$-alkoxycarbonylgruppe bedeutet, $R_2$ für Wasserstoff, $C_1$-$C_4$-Alkyl, N-$C_1$-$C_4$-Alkylamino-$C_2$-$C_7$-alkyl oder N,N-Di-$C_1$-$C_4$-alkylamino-$C_1$-$C_7$-alkyl steht, einer der Reste $R_3$ und $R_4$ Wasserstoff, $C_1$-$C_4$-Alkyl oder Halogen der Atomnummer bis und mit 35 und der andere Wasserstoff darstellt und $R_5$ Halogen der Atomnummer bis und mit 35 bedeutet, mit der Massgabe, dass in Verbindungen der Formel I, in denen $R_5$ für Chlor steht und (a) $R_1$ Acetyl und $R_2$ Wasserstoff bedeuten, oder (b) $R_1$ und $R_2$ beide Methyl bedeuten, mindestens einer der Reste $R_3$ und $R_4$ von Wasserstoff verschieden ist, oder ein Salz davon.

4. Eine Verbindung gemäss Anspruch 1 der Formel I, worin $R_1$ Wasserstoff, $C_1$-$C_4$-Alkyl oder $C_2$-$C_7$-Alkanoyl bedeutet, $R_2$ für Wasserstoff oder $C_1$-$C_4$-Alkyl steht, einer der Reste $R_3$ und $R_4$ Wasserstoff, $C_1$-$C_4$-Alkyl oder Halogen der Atomnummer bis und mit 35 und der andere Wasserstoff darstellt und $R_5$ Halogen der Atomnummer bis und mit 35, mit der Massgabe, dass in Verbindungen der Formel I, in denen $R_5$ für Chlor steht und (a) $R_1$ Acetyl und $R_2$ Wasserstoff bedeuten, oder (b) $R_1$ und $R_2$ beide Methyl bedeuten, mindestens einer der Reste $R_3$ und $R_4$ von Wasserstoff verschieden ist, oder ein Salz davon.

5. Eine Verbindung gemäss Anspruch 1 der Formel I, worin $R_1$ Wasserstoff, $C_2$-$C_7$-Alkanoyl, $C_1$-$C_4$-Alkoxycarbonyl, $C_3$-$C_5$-Alkenyloxycarbonyl, Phenoxycarbonyl oder Phenyl-$C_1$-$C_4$-alkoxycarbonyl bedeutet, $R_2$ für Wasserstoff, $C_1$-$C_4$-Alkyl oder N,N-Di-$C_1$-$C_4$-alkylamino-$C_1$-$C_7$-alkyl steht, einer der Reste $R_3$ und $R_4$ Wasserstoff, $C_1$-$C_4$-Alkyl oder Halogen der Atomnummer bis und mit 35 und der andere Wasserstoff darstellt und $R_5$ Halogen der Atomnummer bis und mit 35 bedeutet, mit der Massgabe, dass in Verbindungen der Formel I, in denen $R_5$ für Chlor steht und $R_1$ Acetyl und $R_2$ Wasserstoff bedeuten, mindestens einer der Reste $R_3$ und $R_4$ von Wasserstoff verschieden ist, oder ein Salz davon.

6. Eine Verbindung gemäss Anspruch 1 der Formel I, worin $R_1$ $C_1$-$C_4$-Alkanoyl und $R_2$ Wasserstoff oder $C_1$-$C_4$-Alkyl bedeutet oder $R_1$ und $R_2$ beide für Wasserstoff stehen, $R_3$ Wasserstoff oder $C_1$-$C_4$-Alkyl und $R_4$ Wasserstoff oder $R_3$ Wasserstoff und $R_4$ Halogen der Atomnummer bis und mit 35 darstellen und $R_5$ Halogen der Atomnummer bis und mit 35 bedeutet, mit der Massgabe, dass in Verbindungen der Formel I, in denen $R_5$ für Chlor steht und $R_1$ Acetyl und $R_2$ Wasserstoff bedeuten, mindestens einer der Reste $R_3$ und $R_4$ von Wasserstoff verschieden ist, oder ein Salz davon.

7. Eine Verbindung gemäss Anspruch 1 der Formel I, worin $R_1$ Wasserstoff oder $C_1$-$C_4$-Alkanoyl bedeutet, $R_2$ Wasserstoff darstellt, $R_3$ $C_1$-$C_4$-Alkyl und $R_4$ Wasserstoff oder $R_3$ Wasserstoff und $R_4$ Halogen der Atomnummer bis und mit 35 darstellen und $R_5$ Halogen der Atomnummer bis und mit 35 bedeutet, mit der Massgabe, dass in Verbindungen der Formel I, in denen $R_5$ für Chlor steht und $R_1$ Acetyl und $R_2$ Wasserstoff bedeuten, mindestens einer der Reste $R_3$ und $R_4$ von Wasserstoff verschieden ist, oder ein Salz davon.

8. Eine Verbindung gemäss Anspruch 1 der Formel I, worin $R_1$ Wasserstoff bedeutet, $R_2$ für Wasserstoff oder $C_1$-$C_4$-Alkyl steht, einer der Reste $R_3$ und $R_4$ Wasserstoff, $C_1$-$C_4$-Alkyl oder Halogen der Atomnummer bis und mit 35 und der andere Wasserstoff darstellt und $R_5$ Halogen der Atomnummer bis und mit 35 bedeutet, oder ein Salz davon.

9. Eine Verbindung gemäss Anspruch 1 der Formel I, worin $R_1$ $C_2$-$C_7$-Alkanoyl bedeutet, $R_2$ für Wasserstoff oder $C_1$-$C_4$-Alkyl steht, $R_3$ Wasserstoff ist, $R_4$ für Wasserstoff oder Halogen der Atomnummer bis und mit 35 steht und $R_5$ Halogen der Atomnummer bis und mit 35 bedeutet, mit der Massgabe, dass in Verbindungen der Formel I, in denen $R_5$ für Chlor steht und $R_1$ Acetyl und $R_2$ Wasserstoff bedeuten, mindestens

einer der Reste $R_3$ und $R_4$ von Wasserstoff verschieden ist, oder ein Salz davon.

**10.** Eine Verbindung gemäss Anspruch 1 der Formel I, worin $R_1$ Wasserstoff bedeutet, $R_2$ für Wasserstoff oder $C_1$-$C_4$-Alkyl steht, $R_3$ Wasserstoff oder $C_1$-$C_4$-Alkyl bedeutet, $R_4$ Wasserstoff, Fluor oder Chlor darstellt und $R_5$ Fluor, Chlor oder Brom bedeutet, oder ein Salz davon.

**11.** Eine Verbindung gemäss Anspruch 1 der Formel I, worin $R_1$ $C_2$-$C_7$-Alkanoyl oder Phenyl-$C_1$-$C_4$-alkoxycarbonyl bedeutet, $R_2$ für Wasserstoff, $C_1$-$C_4$-Alkyl oder N,N-Di-$C_1$-$C_4$-alkylamino-$C_1$-$C_7$-alkyl steht, $R_2$ für Wasserstoff oder $C_1$-$C_4$-Alkyl steht, $R_3$ und $R_4$ für Wasserstoff stehen und $R_5$ Chlor bedeutet, mit der Massgabe, dass in Verbindungen der Formel I, in denen $R_5$ für Chlor steht und $R_1$ Acetyl und $R_2$ Wasserstoff bedeuten, mindestens einer der Reste $R_3$ und $R_4$ von Wasserstoff verschieden ist, oder ein Salz davon.

**12.** Eine Verbindung gemäss Anspruch 1 der Formel I, worin $R_1$ $C_2$-$C_7$-Alkanoyl bedeutet, $R_2$ für Wasserstoff oder $C_1$-$C_4$-Alkyl steht, $R_3$ und $R_4$ für Wasserstoff stehen und $R_5$ Chlor bedeutet, mit der Massgabe, dass in Verbindungen der Formel I, in denen $R_5$ für Chlor steht und $R_1$ Acetyl und $R_2$ Wasserstoff bedeuten, mindestens einer der Reste $R_3$ und $R_4$ von Wasserstoff verschieden ist, oder ein Salz davon.

**13.** Eine Verbindung gemäss Anspruch 1 der Formel I, worin $R_1$ Wasserstoff bedeutet, $R_2$ für Wasserstoff oder $C_1$-$C_4$-Alkyl steht, $R_3$ Wasserstoff und $R_4$ Fluor oder Chlor oder $R_3$ Methyl und $R_4$ Wasserstoff darstellt und $R_5$ Fluor, Chlor oder Brom bedeutet, oder ein Salz davon.

**14.** 1-[4-(N-Propionylamino)benzoyl]-4-[2-(4-chlorphenyl)ethyl]-piperazin gemäss Anspruch 1 oder ein Salz davon.

**15.** 1-(4-Aminobenzoyl)-4-[2-(4-chlorphenyl)ethyl]-piperazin gemäss Anspruch 1 oder ein Salz davon.

**16.** 1-[4-(N-Methylamino)benzoyl]-4-[2-(4-bromphenyl)ethyl]-piperazin gemäss Anspruch 1 oder ein Salz davon.

**17.** Pharmazeutische Präparate enthaltend eine Verbindung gemäss einem der Ansprüche 1 bis 16 und mindestens ein pharmazeutisch verwendbares Trägermaterial.

**18.** Eine Verbindung gemäss einem der Ansprüche 1 bis 16 zur Anwendung in einem Verfahren zur therapeutischen Behandlung des tierischen oder menschlichen Körpers.

**19.** Eine Verbindung gemäss einem der Ansprüche 1 bis 16 zur Verwendung bei der Behandlung von Krankheiten, bei denen eine übermässige Produktion von IL-1 durch monozytische bzw. makrophage Zellinien eine ursächliche oder verschlimmernde Rolle spielt.

**20.** Verwendung einer Verbindung gemäss einem der Ansprüche 1 bis 16 zur Herstellung pharmazeutischer Präparate.

**21.** Verwendung einer Verbindung gemäss einem der Ansprüche 1 bis 16 zur Herstellung pharmazeutischer Präparate für die Behandlung von krankheiten, bei denen eine übermässibe Produktion von IL-1 durch monozytische bzw. makrophage Zellinien eine ursächliche oder verschlimmernde Rolle spielt.

**22.** Verfahren zur Herstellung von Verbindungen der Formel I gemäss Anspruch 1, oder Salzen davon, dadurch gekennzeichnet, dass man

a) eine Verbindung der Formel II,

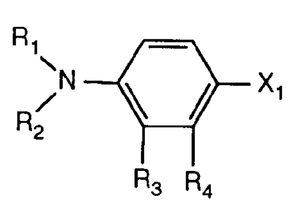

(II),

worin $X_1$ Carboxy oder reaktionsfähiges funktionell abbewandeltes Carboxy bedeutet, oder ein Salz davon, mit einer Verbindung der Formel III,

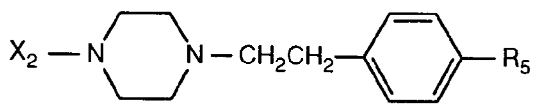

(III),

worin $X_2$ Wasserstoff oder eine Aminoschutzgruppe bedeutet, umsetzt, oder
b) eine Verbindung der Formel IV,

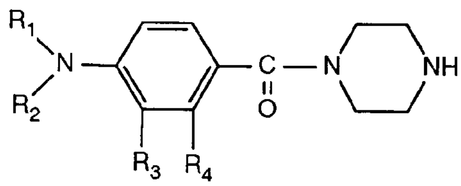

(IV)

oder ein Salz davon, mit einer Verbindung der Formel V,

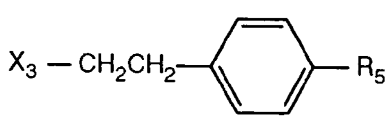

(V),

worin $X_3$ Hydroxy oder reaktionsfähiges verestertes Hydroxy bedeutet, umsetzt, oder
c) eine Verbindung der Formel VI,

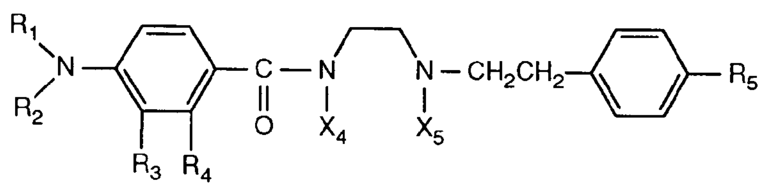

(VI)

worin einer der Reste $X_4$ und $X_5$ Wasserstoff und der andere eine Gruppe der Formel $-CH_2-CH_2-X_3$ (VIa) bedeutet, und $X_3$ für Hydroxy oder reaktionsfähiges verestertes Hydroxy steht, cyclisiert, oder
d) eine Verbindung der Formel VII,

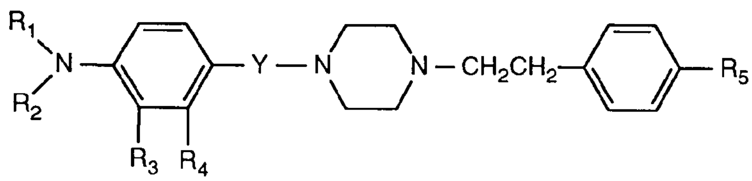

(VII),

worin Y eine zu Carbonyl oxidierbare Gruppe bedeutet, oxidiert und gewünschtenfalls eine verfahrensgemäss oder auf andere Weise erhältliche Verbindung in eine andere Verbindung der Formel I überführt, ein verfahrensgemäss erhältliches Isomerengemisch in die Komponenten auftrennt, eine verfahrensgemäss erhältliche freie Verbindung der Formel I in ein Salz überführt und/oder ein verfahrensmäss erhältliches Salz in die freie Verbindung der Formel I oder in ein anderes Salz überführt.

Europäisches Patentamt

# EUROPÄISCHER RECHERCHENBERICHT

Nummer der Anmeldung

EP 91 81 0926

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | EP-A-0 250 361 (CIBA-GEIGY AG.)<br>* Ansprüche; Beispiele 28-29 *<br>--- | 1-22 | C07D295/18<br>A61K31/495 |
| A | GB-A-874 096 (SOCIETE DES USINES CHIMIQUES RHONE-POULENC)<br>* Ansprüche; Beispiele *<br>----- | 1 | |
| | | | **RECHERCHIERTE SACHGEBIETE (Int. Cl.5 )** |
| | | | C07D |

Der vorliegende Recherchenbericht wurde für alle Patentansprüche erstellt

| Recherchenort | Abschlußdatum der Recherche | Prüfer |
|---|---|---|
| DEN HAAG | 18 FEBRUAR 1992 | PAUWELS G.R.A. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)